(19) Europäisches Patentamt
European Patent Office
Office européen des brevets

(11) EP 0 907 684 B1

(12) **FASCICULE DE BREVET EUROPEEN**

(45) Date de publication et mention de la délivrance du brevet:
**27.03.2002 Bulletin 2002/13**

(21) Numéro de dépôt: **97930600.8**

(22) Date de dépôt: **26.06.1997**

(51) Int Cl.[7]: **C08L 83/04**, C08G 77/38, G02B 1/04, C07C 49/796, C07C 49/84

(86) Numéro de dépôt international:
**PCT/FR97/01147**

(87) Numéro de publication internationale:
**WO 97/49768 (31.12.1997 Gazette 1997/57)**

(54) **MATERIAU A BASE DE SILICONE RETICULE COMPORTANT UN PHOTOAMORCEUR FIXE, SON PROCEDE DE PREPARATION, PRODUIT POLYMERIQUE HYDROPHILE OBTENU A PARTIR DE CE MATERIAU ET SON PROCEDE DE PREPARATION, ET NOUVEAUX PHOTOAMORCEURS**

GEBUNDENE PHOTOKATALYSATOREN ENTHALTENDE, GEHÄRTETE SILIKONZUSAMMENSETZUNG, VERFAHREN ZU DEREN HERSTELLUNG, HYDROPHILE POLYMERZUSAMMENSETZUNGEN, DEREN HERSTELLUNG UND NEUE PHOTOKATALYSATOREN

CROSS-LINKED SILICONE BASED MATERIAL COMPRISING A BOUND PHOTOINITIATOR, ITS METHOD OF PREPARATION, HYDROPHILIC POLYMERIC PRODUCT OBTAINED FROM THIS MATERIAL AND ITS METHOD OF PREPARATION, AND NOVEL PHOTOINITIATORS

(84) Etats contractants désignés:
**AT BE CH DE DK ES FR GB IT LI SE**

(30) Priorité: **27.06.1996 FR 9608031**

(43) Date de publication de la demande:
**14.04.1999 Bulletin 1999/15**

(73) Titulaire: **ESSILOR INTERNATIONAL COMPAGNIE GENERALE D'OPTIQUE**
**94227 Charenton cédex (FR)**

(72) Inventeurs:
- **MARCHIN, Brigitte**
  **F-94220 Charenton-le-Pont (FR)**
- **BAUDE, Dominique**
  **F-93400 Saint-Ouen (FR)**
- **VAIRON, Jean-Pierre**
  **F-92340 Bourg-la-Reine (FR)**
- **DOURGES, Marine-Anne**
  **F-95130 Franconville (FR)**
- **CHAUMONT, Philippe**
  **F-69006 Lyon (FR)**
- **STEINER, Jo[l**
  **F-67300 Schiltigeim (FR)**

(74) Mandataire: **Catherine, Alain et al**
**Cabinet Harlé & Phélip**
**7, rue de Madrid**
**75008 Paris (FR)**

(56) Documents cités:

| | |
|---|---|
| **EP-A- 0 088 842** | **EP-A- 0 108 037** |
| **US-A- 4 362 895** | **US-A- 4 534 838** |
| **US-A- 4 536 265** | **US-A- 4 921 589** |

- **PATENT ABSTRACTS OF JAPAN vol. 006, no. 227 (C-134), 12 novembre 1982 & JP 57 131230 A (TORAY SILICONE KK), 14 août 1982,**
- **DATABASE BEILSTEIN BEILSTEIN INSTITUTE FOR ORGANIC CHEMISTRY, FRANKFURT/MAIN, DE brn=4669833, XP002028694 & YOKOYAMA YUUSAKU ET AL.: TETRAHEDRON LETTERS, vol. 26, no. 52, 1985, OXFORD GB, pages 6457-6460,**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

L'invention concerne un procédé de préparation de produits polymériques hydrophiles obtenus à partir d'un matériau à base de polymère de silicone, comportant une matrice de polymère de silicone réticulé et des groupements photoamorceurs dispersés et fixés au sein de la matrice de silicone, et leur application à la fabrication de lentilles de contact hydrophiles.

L'invention concerne également de nouveaux photoamorceurs spécialement adaptés pour être incorporés dans le matériau à base de polymère de silicone réticulé ci-dessus et pour la mise en oeuvre du procédé conduisant aux produits polymériques hydrophiles.

Les matériaux à base de silicone ou polysiloxanes sont bien connus pour leur très haute perméabilité à l'oxygène, en particulier les polydiméthylsiloxanes (PDMS). L'utilisation des polysiloxanes purs n'est cependant pas envisageable pour la réalisation de lentilles de contact car ce matériau possède l'inconvénient d'être hydrophobe et présente donc une absence de mouillabilité de surface qui provoque une rupture du film lacrymal. Par ailleurs, des lentilles de contact en silicone pur provoquent des effets de ventouse (adhésion sur la cornée de l'oeil).

Diverses techniques ont déjà été proposées pour rendre les lentilles de silicone compatibles avec l'oeil.

Certaines techniques visent à traiter la surface de la lentille pour la rendre hydrophile.

On connaît, par exemple, dans le brevet français FR 2.073.034, un procédé pour rendre hydrophile en surface des lentilles de contact en silicone qui consiste à faire gonfler la matrice de silicone à l'aide d'un monomère de type monoester ou monoamide de l'acide acrylique ou méthacrylique formant un polymère hydrophile.

Les lentilles de contact ainsi obtenues présentent un taux très faible de polymère acrylique ou méthacrylique incorporé à la surface de la matrice de silicone.

D'autres techniques visent à obtenir des matériaux hydrophiles du type à réseau interpénétré (IPN) à partir de prépolymères hydrophobes d'organosiloxanes et de monomères hydrophiles du type monoester ou monoamide d'acide acrylique ou méthacrylique. Le matériau de type IPN est un matériau dans lequel les prépolymères hydrophobes et les monomères hydrophiles sont mélangés ensemble et réagissent simultanément pour former deux réseaux imbriqués par une polymérisation indépendante des prépolymères hydrophobes et des monomères hydrophiles, respectivement.

Cette technique présente des inconvénients car il existe des problèmes de solubilité du monomère hydrophile dans le prépolymère hydrophobe. Il est donc difficile d'obtenir des matériaux homogènes et cette technique n'est pas adaptée pour obtenir des matériaux à concentration élevée en polymère hydrophile et donc à des teneurs élevées en eau dans le matériau final.

Le brevet français FR 2709756 au nom de la demanderesse décrit un procédé d'obtention d'un matériau silicone hydrophile de type IPN consistant, dans une première étape, à faire gonfler un polymère de type PDMS dans une composition renfermant de l'acide acrylique, un photoamorceur, un agent de réticulation et un solvant de gonflement du polymère PDMS, puis à provoquer la polymérisation de l'acide acrylique en soumettant le polymère PDMS gonflé à une irradiation UV. Il est ainsi possible d'obtenir des matériaux hydrophiles à coeur, avec des taux d'hydrophilie élevés. Bien que ce procédé donne des résultats satisfaisants, il reste cependant perfectible. En particulier, il existe une possibilité d'hétérogénéité dans la répartition photoamorceur/monomère hydrophile au sein de la matrice, dans la mesure où ces composés diffusent à des vitesses différentes, ce qui peut conduire à des hétérogénéités physiques et mécaniques.

Il serait par ailleurs souhaitable d'augmenter encore le taux d'hydrophilie du matériau final.

Enfin, une simplification dans la mise en oeuvre du procédé décrit dans le brevet français FR 2.709.756 est également souhaitable pour son exploitation industrielle et pour son application à la fabrication de lentilles de contact.

Par ailleurs, le brevet US-4.921.589 décrit un photosensibilisateur lié à un polymère de polysiloxane et son utilisation dans un procédé d'oxydation des composés oxydables indésirables présents dans une fraction hydrocarbure ou aqueuse.

Afin de résoudre ces problèmes techniques, on a réalisé un matériau à base de polymère de silicone réticulé comportant une matrice de polymère de silicone réticulé, c'est-à-dire dont le réseau polymère est tridimensionnel, et dont la caractéristique est de comporter des groupements photoamorceurs dispersés et fixés au sein de la matrice de silicone.

Les procédés de traitement ultérieur de ce matériau à base de polymère de silicone réticulé visant à le rendre hydrophile à coeur s'en trouvent grandement simplifiés et les produits hydrophiles obtenus présentent des caractéristiques particulièrement intéressantes et avantageuses.

Selon l'invention, le procédé de fabrication d'un produit polymérique hydrophile consiste à :

(a) faire gonfler un matériau comprenant une matrice de polymère de silicone réticulé et des groupements photoamorceurs dispersés et fixés au sein de la matrice dans une solution de gonflement comprenant un solvant de gonflement du polymère de silicone réticulé de la matrice du matériau, un monomère hydrophile photopolymérisable, et éventuellement :

1. un agent de réticulation ; et
2. un composé coamorceur donneur de protons ou d'électrons lorsque le matériau à base de polymère de silicone comprend des groupements photoamorceurs photoactivables et ne comprend pas de groupements coamorceurs donneurs de protons ou d'électrons ;

(b) faire diffuser le monomère hydrophile photopolymérisable dans le matériau gonflé ; et
(c) polymériser par irradiation le monomère hydrophile photopolymérisable.

On va maintenant décrire plus en détail le matériau à base de polymère de silicone réticulé comportant des groupements amorceurs fixés et son procédé de préparation.
Selon l'invention, la matrice de silicone réticulée comporte des groupements ou fragments photoamorceurs répartis de façon homogène dans tout son volume et jusqu'au coeur même de la matrice.
Les groupements photoamorceurs peuvent être fixés par les techniques suivantes :
Selon une première technique, les groupements photoamorceurs sont fixés à la matrice de silicone par une liaison chimique covalente.
A cet effet, on prépare un photoamorceur fonctionnalisé par un groupement silyle SiH ou par une double liaison C = C insaturée.
Cette double liaison peut être de type vinylique, (meth)acrylique ou allylique.
Le composé photoamorceur est introduit dans une composition liquide réticulable comprenant :

- une huile A d'un monomère ou d'un oligomère de polysiloxane porteur de groupements Si-Vinyle ;
- une huile B d'un monomère ou d'un oligomère de polysiloxane porteur de groupements Si-H ;
- un catalyseur métallique pour la réaction d'hydrosilylation.

Lors de la réticulation effectuée par voie thermique, par réaction d'hydrosilylation, le composé photoamorceur se greffe sur le réseau polysiloxanique par l'intermédiaire d'une liaison covalente Si-C.
Selon une seconde technique de fixation, on utilise un composé photoamorceur à longue chaîne. Cette chaîne est de préférence une chaîne polysiloxane sur laquelle le ou les groupement(s) photoamorceur(s) est (sont) greffé(s). Ce composé photoamorceur est, de la même façon que dans la technique précédente, introduit dans le mélange liquide des précurseurs polysiloxaniques.
Lors de la réticulation du mélange, la chaîne polysiloxanique du composé photoamorceur se trouve physiquement immobilisée au sein de la matrice de polymère de silicone réticulé obtenue.
Il n'existe pas dans ce cas, contrairement à la technique précédente, de liaison chimique entre le composé photoamorceur et la matrice de silicone, mais une simple rétention de nature physique, due en particulier au caractère tridimensionnel du réseau du polymère de silicone réticulé de la matrice.
Cependant, quelle que soit la technique de fixation utilisée, les groupements photoamorceurs restent fixés dans la matrice de polysiloxane même lorsque celle-ci est soumise à des traitements d'extraction aux solvants. En d'autres termes, il n'est pas possible de séparer par une extraction avec des solvants les groupements photoamorceurs de la matrice, que ceux-ci soient greffés au polymère formant la matrice ou simplement portés par un composé à longue chaîne immobilisé physiquement au sein de la matrice.
Ainsi donc, en fin de réaction d'hydrosilylation, on obtient un matériau à base de polymère de silicone réticulé comportant une matrice de polymère de silicone réticulé dans laquelle sont répartis des groupements photoamorceurs aptes à engendrer des radicaux libres par irradiation lumineuse.
Les compositions de silicones liquides utilisées pour l'obtention de la matrice de polymère de silicone sont de préférence des huiles de polydiméthylsiloxanes à deux constituants dont les motifs constitutifs essentiels sont représentés ci-après :

Constituant A 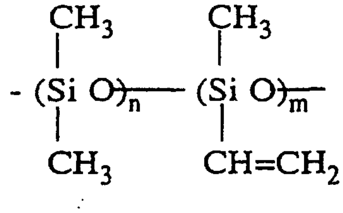

Constituant B 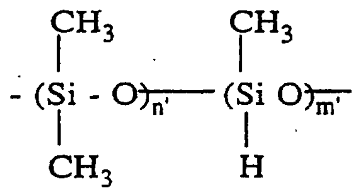

Ces huiles de silicone sont réticulées grâce à un catalyseur d'hydrosilylation. De tels catalyseurs sont bien connus de l'homme du métier.

Il s'agit, par exemple du platine, de l'acide hexachloroplatinique, de complexes platine-hydrocarbone, et de complexes de rhodium.

Les catalyseurs à base de platine sont généralement utilisés à des concentrations de 10 ppm à 500 ppm, préférentiellement de 50 à 300 ppm.

Les températures de réaction varient de la température ambiante à 250°C en fonction de la concentration et du type de catalyseur utilisé. Les températures préférentielles varient de 50°C à 150°C.

Les constituants A et B sont utilisés dans des proportions telles que leur mélange renferme de 0,8 à 1,9 liaisons SiH pour 1 liaison Si-Vinyle.

De préférence, le polymère de polydiméthylsiloxane (PDMS) est préparé en mélangeant deux prépolymères siloxaniques développés par Rhône-Poulenc sous la référence RTV 141 A et B et le photoamorceur fonctionnalisé dans les proportions souhaitées.

L'huile A est constituée de PDMS mono- et divinyliques et d'un catalyseur platinique. Cette partie contient environ $3,10 \times 10^{-4}$ fonctions vinyle par gramme de RTV 141 A et sa masse moléculaire moyenne en nombre est 31200.

L'huile B est de préférence un hydrogénométhyle PDMS et contient $4,07 \times 10^{-3}$ fonctions SiH par gramme de RTV 141 B et sa masse moléculaire moyenne en nombre est 1770.

Pour l'obtention du polymère, on mélange 10 parties en poids d'huile A et 1 partie en poids d'huile B et le photoamorceur, puis on procède à la réticulation comme mentionnée précédemment.

On va maintenant décrire plus en détail les photoamorceurs au moyen desquels on peut introduire les groupements photoamorceurs dans les compositions liquides réticulables décrites ci-dessus.

Les composés photoamorceurs comportent, d'une part, un groupement fonctionnel destiné à réagir avec les groupements SiH ou Si-vinyles des huiles de PDMS, et, d'autre part, un groupement photoamorceur.

De tels composés peuvent être obtenus en fonctionnalisant des photoamorceurs classiques, à savoir : tout composé producteur de radicaux libres sous irradiation, que ce soit par lui-même ou par coopération avec un autre composé donneur de protons ou d'électrons. C'est dire que les photoamorceurs utilisés, ou amorceurs de photopolymérisation, peuvent être aussi bien de type photoclivables que de type photo-activables, avec toutefois une préférence pour ceux qui sont actifs pour amorcer la photopolymérisation du monomère pour des longueurs d'onde d'irradiation se situant dans le domaine de l'UV.

Un photoamorceur photoclivable comporte un ou plusieurs composés qui fonctionnent en générant directement un ou plusieurs radicaux libres amorceurs de polymérisation, tandis qu'un photoamorceur photoactivable est formé d'un système produisant de tels radicaux par une réaction d'oxydoréduction photo-assistée entre un composé absorbeur de lumière et un donneur d'hydrogène ou d'électrons tous deux présents dans le système. Bien entendu, on peut également utiliser des mélanges des deux types de photoamorceurs.

Des exemples de composés photoclivables connus en soi sont choisis parmi les dérivés d'alcoxyacétophénone, éthers de benzoïne, phosphine-oxydes, dérivés de benzoyloxime. Des exemples de photoamorceurs photoactivables connus comprennent un absorbeur producteur de radicaux libres choisi parmi les benzophénones, benzyles, xanthones, anthrones, thioxanthones, fluorénones, subérones, acridones, en association avec, comme donneur de protons, un composé du type des éthers, alcools, amines, amino-acides, ou composés oraganométalliques.

De tels photoamorceurs peuvent être fonctionnalisés par des techniques connues de l'homme de l'art.

On pourra utilement se référer à l'enseignement des documents suivants :

- le brevet US 4507187 décrivant la fabrication d'un photoamorceur de type formiate d'aryoyle fonctionnalisé par des groupements alcéniques ou acétyléniques;
- les brevets US 4477326 et US 4587276 décrivant l'obtention de photoamorceurs de type benzoïne fonctionnalisés par des groupements allyliques;
- le brevet US 4536265 décrivant l'obtention d'acétophénones à fonctionnalité oléfinique ou acétylénique; et
- le document "Photoinitiator with functional group" J.M.S - Pure Appl - Chem A 31 (3) pp 305-318 (1994) - KOLAR,

GRUBE, GREBER, qui décrit des photoamorceurs fonctionnalisés par un groupe silyle, à savoir la 2-hydroxy (ou 2-méthoxy)-2-méthyl-1-(4-diméthylsilyl)phényl-propane-1-one.

Parmi les photoamorceurs utiles dans la présente invention, on recommande plus particulièrement ceux répondant à la formule :

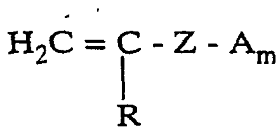 (I)

dans laquelle :

R représente un atome d'hydrogène ou un groupe méthyle,
Z est une chaîne hydrocarbonée divalente comportant de 1 à 10 atomes de carbone, qui peut être interrompue par 1 à 3 atomes choisis parmi -O-.

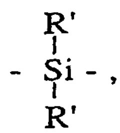

où R' est indépendamment un atome d'hydrogène ou un groupe alkyle, de préférence un groupe alkyle en $C_1$-$C_6$, et mieux encore un groupe méthyle, et
$A_m$ est un groupement comportant une fonction

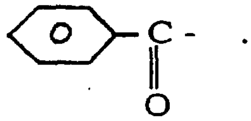

De préférence Z représente les chaînes divalentes suivantes :

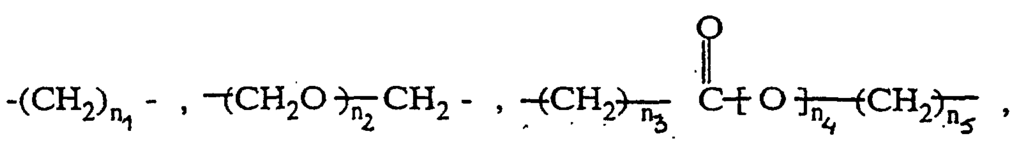

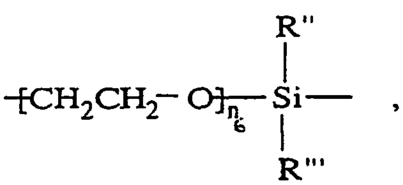

dans lesquelles R'' et R''' représentent indépendamment l'un de l'autre un groupe alkyle, de préférence en $C_1$-$C_6$, et en particulier un groupe méthyle, $n_1$ et $n_2$ sont des entiers de 1 à 6, $n_3$ et $n_5$ sont des entiers de 0 à 4, $n_4$ est égal à 0 ou 1, et $n_6$ est un entier de 0 à 5.

De préférence, le groupement $A_m$ est choisi parmi les groupements répondant aux formules :

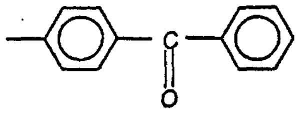 (II),

à condition que Z comporte au moins deux atomes de carbone ;

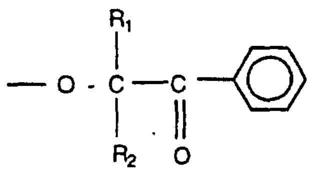 (III) ;

et

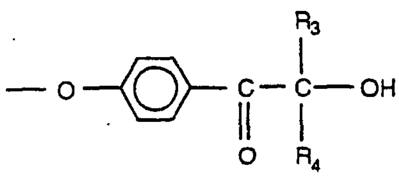 (IV) ;

dans lesquelles $R_1$, $R_2$, $R_3$ et $R_4$, identiques ou différents, sont choisis parmi l'hydrogène et les groupements alkyle ayant de 1 à 6 atomes de carbone, de préférence un groupement méthyle.

D'autres photoamorceurs utiles dans la présente invention répondent aux formules suivantes :

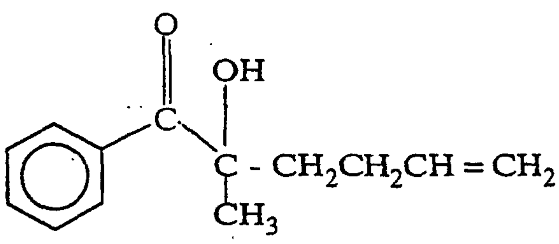 (Va)

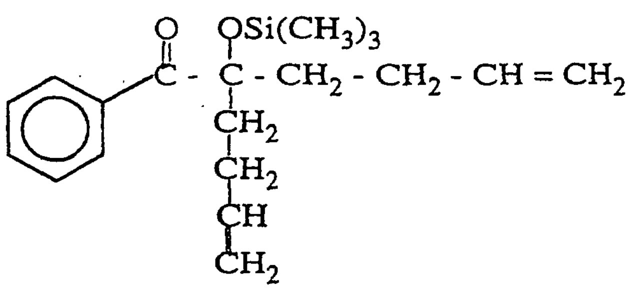 (Vb)

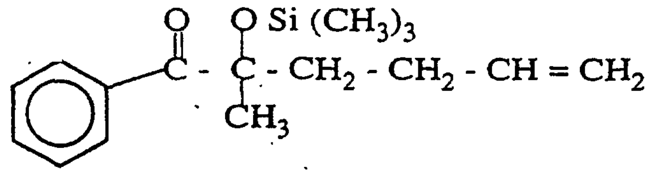

(Vc)

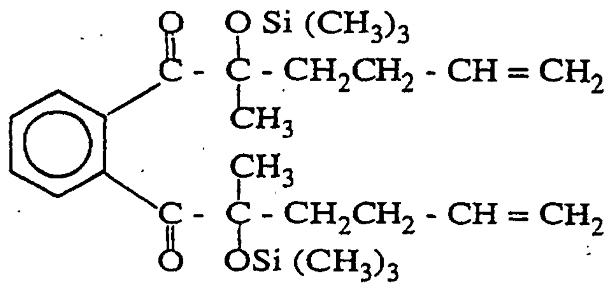

(Vd)

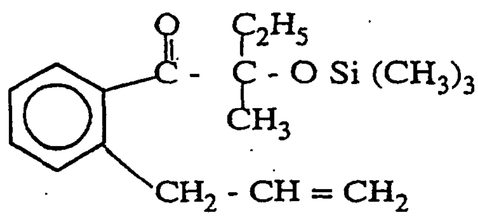

(Ve)

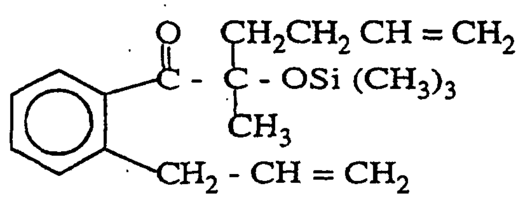

(Vf)

Les photoamorceurs répondant à la formule (I) ci-dessus et dont le groupement $A_m$ est choisi parmi les groupements de formules (II) à (IV) sont nouveaux ainsi que les composés de formules (Vb) à (Vf).

La présente invention concerne donc également ces nouveaux photoamorceurs spécialement adaptés pour être incorporés dans le matériau à base de polymère de silicone réticulé, selon l'invention, ainsi que pour la mise en oeuvre du procédé conduisant aux produits polymériques hydrophiles de la présente invention.

Préférentiellement, les photoamorceurs répondent à l'une des formules suivantes :

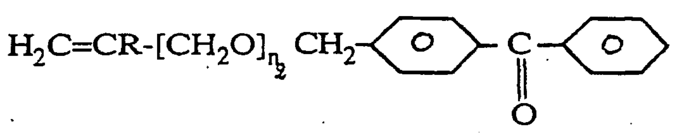

(VI)

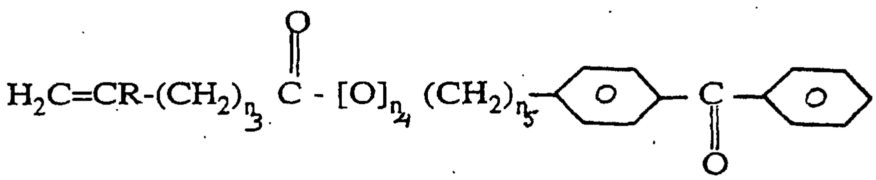

(VII)

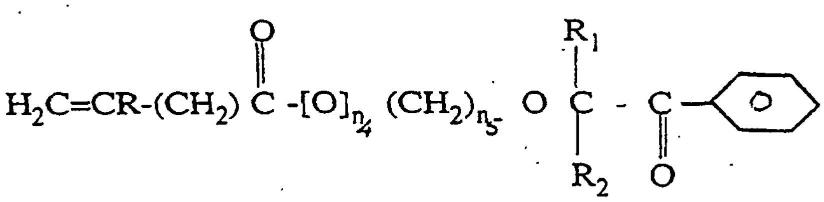

(VIII)

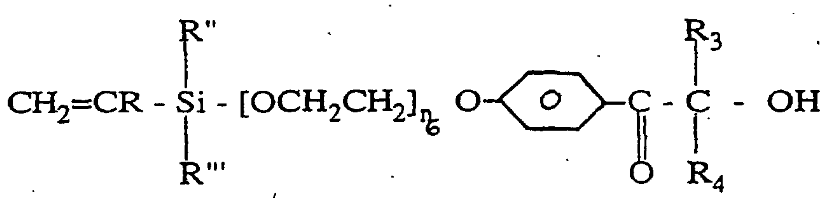

(IX)

dans lesquelles R, R", R"', $R^1$, $R^2$, $R^3$, $R^4$,
$n_2$, $n_3$, $n_4$, $n_5$ et $n_6$ ont les mêmes significations que précédemment.

Les photoamorceurs utilisés préférentiellement dans le cadre de l'invention sont :

l'allyl-4 benzophénone (ALBP)

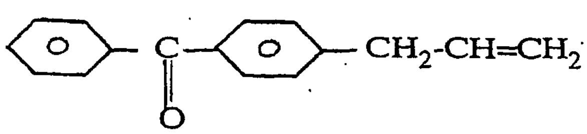

l'allyloxyméthyl-4 benzophénone (ALOBP)

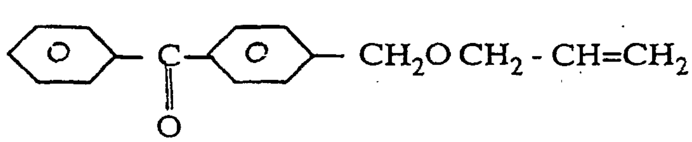

De tels photoamorceurs sont introduits comme mentionnés précédemment dans le mélange des prépolymères siloxaniques à raison de plus de 0,1% en poids, de préférence 0,5 à 10% en poids et mieux encore de 0,5 à 4% en poids.

Le photoamorceur sera ainsi fixé par liaison covalente au silicone final.

On peut également utiliser dès photoamorceurs à longue chaîne obtenus en faisant réagir l'un des photoamorceurs mentionnés précédemment avec une huile polysiloxanique, de préférence à chaîne linéaire, par réaction d'hydrosilylation. De préférence, l'huile polysiloxanique est une huile PDMS dont la masse moléculaire est comprise entre 132 et 50 000 g/mole, préférentiellement entre 250 et 10 000 g/mole.

L'obtention de tels photoamorceurs est décrite dans l'article de KOLAR, GRUBE, GREBER, mentionné précédemment, de même que dans l'article "Functional polysiloxanes with benzophenone side groups : a photochemical

application as radical polymerisation macro-initiators Lydie Pouliquen, Xavier Coqueret, Alain Lablache-Combier, Claude Loucheux. Makromol. Chem. 113, 1273-1282 (1992).

Le photoamorceur à longue chaîne est introduit dans l'huile PDMS à une concentration telle que la fraction en poids des groupements fonctionnels photoamorçants soit égale à celle utilisée pour les photoamorceurs classiques, typiquement de 0,05 à 5% en poids de groupements fonctionnels photoamorceurs préférentiellement de 0,05 à 2% en poids.

Lorsqu'on utilise des photoamorceurs photoactivables, le composé donneur d'hydrogène ou d'électrons peut être introduit dans la solution de gonflement utilisée dans la première étape du procédé d'hydrophilisation.

Le composé donneur peut être également fixé au réseau de la matrice de PDMS, c'est-à-dire introduit dans le mélange des prépolymères siloxaniques, dans la mesure où le composé donneur a été préalablement fonctionnalisé.

Dans ce cas, le composé donneur sera fixé au réseau de la matrice de silicone par une liaison covalente.

On peut également fixer ce composé donneur fonctionnalisé sur une chaîne polysiloxanique par réaction d'hydrosilylation et obtenir ainsi un composé donneur à longue chaîne polysiloxanique, de préférence PDMS, qui, introduit dans le mélange des prépolymères siloxaniques, sera physiquement retenu dans la matrice de silicone finale. Pour la préparation du composé donneur à longue chaîne polysiloxanique, on utilise la même huile polysiloxanique que celle définie pour la préparation du composé photoamorceur à longue chaîne polysiloxanique.

Un exemple de composé donneur ou coamorceur est le diméthyl-amino-4 benzoate d'éthyle (DMABE)

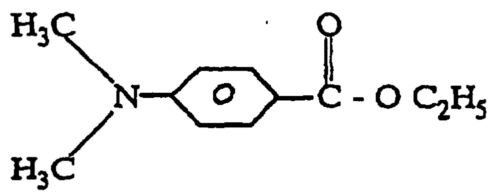

Un coamorceur fonctionnalisé utilisable est la 4-diméthylvinylsilane-N,N-diméthylaniline.

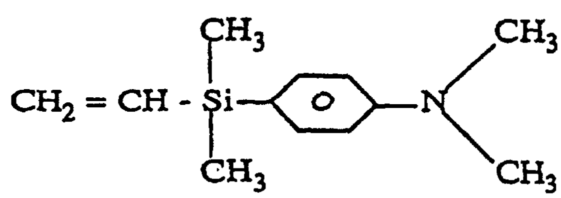

De manière générale, la quantité de coamorceur utilisée est fonction de la quantité de photoamorceur utilisée. On utilise des concentrations en unités coamorçantes choisies dans les mêmes gammes de concentration que les photoamorceurs.

Les photoamorceurs ci-dessus peuvent être préparés par des procédés classiques bien connus de l'homme du métier.

On a indiqué ci-après des exemples de préparation de photoamorceurs.

Synthèse de 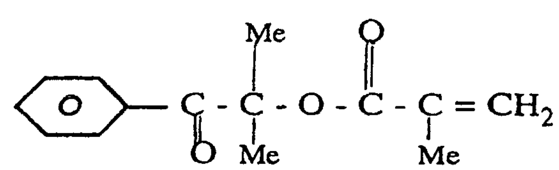 (méthacrylate de DAROCUR®)

On introduit dans un ballon à trois cols de 250 ml muni d'unthermomètre, d'une entrée d'argon et d'une burette d'introduction 20 g

(0,122 mole) de DAROCUR ® 1173 ( 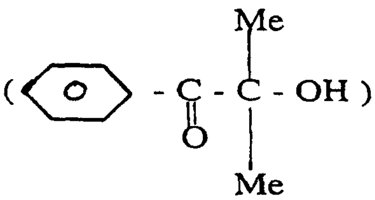

On ajoute 250 ml d'éther distillé une fois sur du sodium et 20,4 ml (0,146 g) de triéthylamine distillée. Après une purge à l'argon, on ajoute goutte à goutte, sous agitation, le chlorure d'acide méthacrylique. La température du milieu réactionnel s'accroît et on refroidit de temps en temps au moyen d'un bain d'eau froide. Le milieu réactionnel se trouble immédiatement, l'opacité augmentant au fur et à mesure de l'addition et s'accentue encore après la fin de l'addition du chlorure d'acide. Une fois tout le chlorure d'acide ajouté (13,1 ml - 0,134 mole), on laisse le mélange réactionnel sous agitation à température ambiante pendant 2 heures. On lave ensuite le mélange obtenu avec 100 ml d'eau additionnée de quelques millilitres de HCl, puis avec 100 ml d'eau et enfin avec 100 ml d'eau additionnée de quelques grammes de $NaHCO_3$. On sèche le produit obtenu sur $MgSO_4$, on filtre et on évapore à sec pour obtenir 23,5 g de produit attendu. (Rendement : 83%).

Le produit obtenu est le méthacrylate de DAROCUR ® de formule :

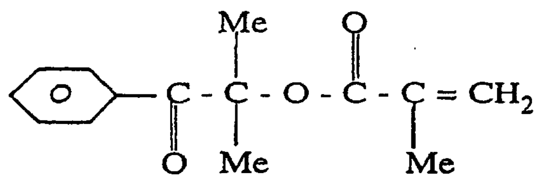

Synthèse de $H_2C = CH (Me)_2 SiO (CH_2)_2$ O 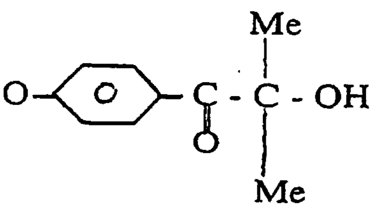

(Vinylsilane d'Irgacure ® ou VSI).

Dans un ballon multicol de 500 ml purgé à l'argon et muni d'une burette d'introduction de tétrahydrofuranne (THF), d'un thermomètre, d'une entrée d'argon, d'une burette d'introduction de triéthylamine (TEA) et d'une burette d'introduction de chlorovinyldiméthylsilane, on introduit 150 ml de THF anhydre, 9,37 g (4,18 x $10^{-2}$ mole)

d'Irgacure® 2959 ($HO(CH_2)_2$ - O 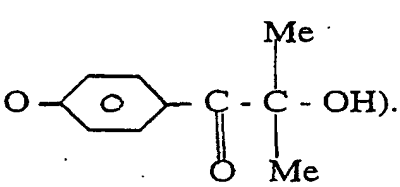

Ce dernier se met rapidement en solution. On ajoute alors 3,82 ml de TEA distillée sur $CaH_2$, et enfin on ajoute goutte à goutte le chlorovinyldiméthylsilane. La température du mélange réactionnel s'élève et on le refroidit au moyen d'un bain de glace. Il se forme immédiatement un précipité blanc crème. A la fin de l'addition du chlorovinyldiméthyl silane (5,04 g- 4,18 x $10^{-2}$ mole), on laisse le mélange réactionnel sous agitation pendant 4 heures. On filtre la solution obtenue sur verre fritté (porosité 4). On chasse du produit filtré le THF et on le reprend avec du cyclohexane. On note l'apparition d'un trouble qui est éliminé par filtration. On chasse alors le solvant pour recueillir 12,44 g d'une huile jaune. Après distillation sous pression réduite et à 175°C, on recueille deux fractions de 5,13 g et 3,27 g, respectivement, du produit attendu, soit un rendement de 65%.

Le produit obtenu est le vinylsilane d'Irgacure® de formule :

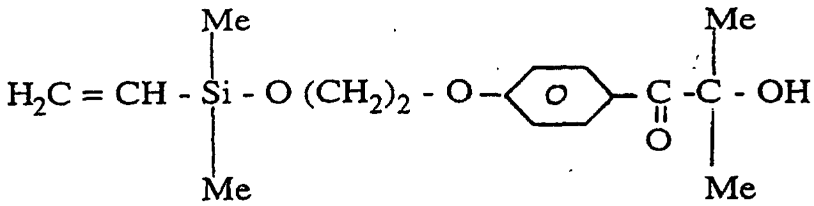

Synthèse de 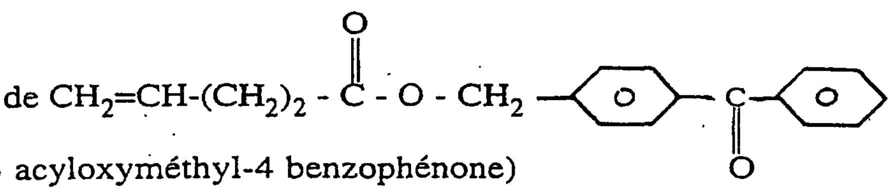

(butène-3 acyloxyméthyl-4 benzophénone)

**a.** Préparation de la 4-bromométhyl benzophénone

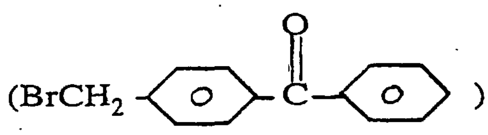

Dans un tricol de 250 ml, équipé d'un réfrigérant et d'une ampoule à brome contenant 8,7 ml (0,168 mole) de brome, on introduit 30 g (0,153 mole) de 4-méthylbenzophénone. Dans un premier temps on chauffe le milieu réactionnel à 70°C puis, après dissolution complète du solide, à 150°C. On ajoute alors le brome goutte à goutte sur une durée de 3 heures. La réaction est exothermique. La solution initialement incolore devient jaune-orange. On maintient le chauffage à 150°C pendant 15 heures. A la fin de la réaction, on laisse la température du milieu réactionnel revenir à la température ambiante. La solution est de couleur brune. On ajoute 75 ml de benzène et 35 ml de diéthyléther. On lave avec une solution de $Na_2CO_3$ saturée, puis avec une solution de $Na_2S_2O_3$ saturée, et enfin avec une solution de NaCl saturée pour éliminer les sels formés et ramener la solution à un pH de l'ordre de 6-7. On récupère la phase organique et on évapore les solvants. On obtient une pâte de couleur caramel. On recristallise dans 100 ml d'éthanol. On recueille les cristaux de couleur crème qui sont séchés sous vide. Les eaux mères sont recueillies, et on effectue une deuxième cristallisation. Les cristaux obtenus sont séchés sous vide. Le rendement en produit attendu est de 50%, m = 20,81 g.

**b.** Synthèse de la 4-hydroxyméthylbenzophénone :

Dans un tricol, muni d'un réfrigérant et sous agitation magnétique, on introduit la 4-bromométhylbenzophénone obtenue à l'étape précédente (2 g, 7,27 mmoles). Dans un erlenmeyer on prépare une solution à 15% en volume d'eau et de N-méthyl-2-pyrrolidone (5,6/31,5 ml). On observe un léger échauffement. On introduit ensuite cette solution dans une ampoule à brome, et on l'ajoute goutte à goutte en 1h45 à la 4-bromométhylbenzophénone tout en chauffant le milieu réactionnel à 80°C. A la fin de l'addition, la température de la solution est élevée progressivement jusqu'à 110°C. On maintient le chauffage pendant 18 heures. A la fin de la réaction, la température du milieu réactionnel revient à l'ambiante. On ajoute 70 ml d'eau, le milieu réactionnel devient blanc après mélange. On extrait la phase organique 3 fois avec 70 ml d'éther diéthylique. Puis on regroupe les phases organiques, on les lave 3 fois avec 150 ml d'eau et on les sèche sur $MgSO_4$. Après évaporation des solvants à l'évaporateur rotatif, on obtient 1,43 g de produit brut

(rendement brut = 93%).

La purification s'effectue par distillation au four à boules. On obtient alors 1,16 g du produit attendu (rendement final = 75%).

**c.** Condensation de la 4-hydroxyméthylbenzophénone sur le chlorure de l'acide pentène-4 oïque :

Dans un ballon on introduit le chlorure de l'acide pentène-4 oïque (0,77 g, 6,5 mmoles) et la 4-hydroxyméthylbenzophénone (1,16 g, 5,5 mmoles) dans 20 ml de THF anhydre. On ajoute goutte à goutte de la pyridine (0,56 g, 7 mmoles). Un précipité blanc se forme au fur et à mesure de l'ajout (sel de pyridinium). La réaction se poursuit pendant 3 heures sous agitation magnétique. A la fin de la réaction, le sel est filtré et lavé avec de l'éther. Puis on récupère le filtrat et on ajoute de l'eau. La phase aqueuse est lavée, puis extraite 3 fois avec 30 ml d'éther. On regroupe les phases organiques, on ajoute une solution aqueuse de $K_2CO_3$ et on ramène la phase organique à neutralité par addition d'une solution aqueuse saturée en NaCl. On sèche ensuite sur $MgSO_4$ et on élimine les solvants. On obtient 1,28 g du produit attendu (rendement = 79%).

Le composé photoamorceur obtenu répond à la formule :

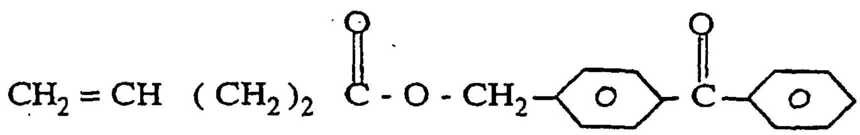

Synthèse de l'allyl-4 benzophénone (ALBP)

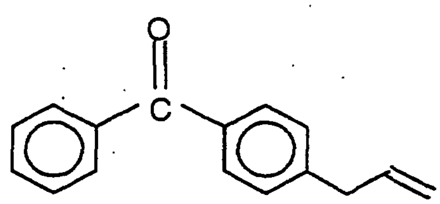

On indique ci-dessous le schéma de préparation de l'allylbenzophénone.

**Schéma de synthèse :**

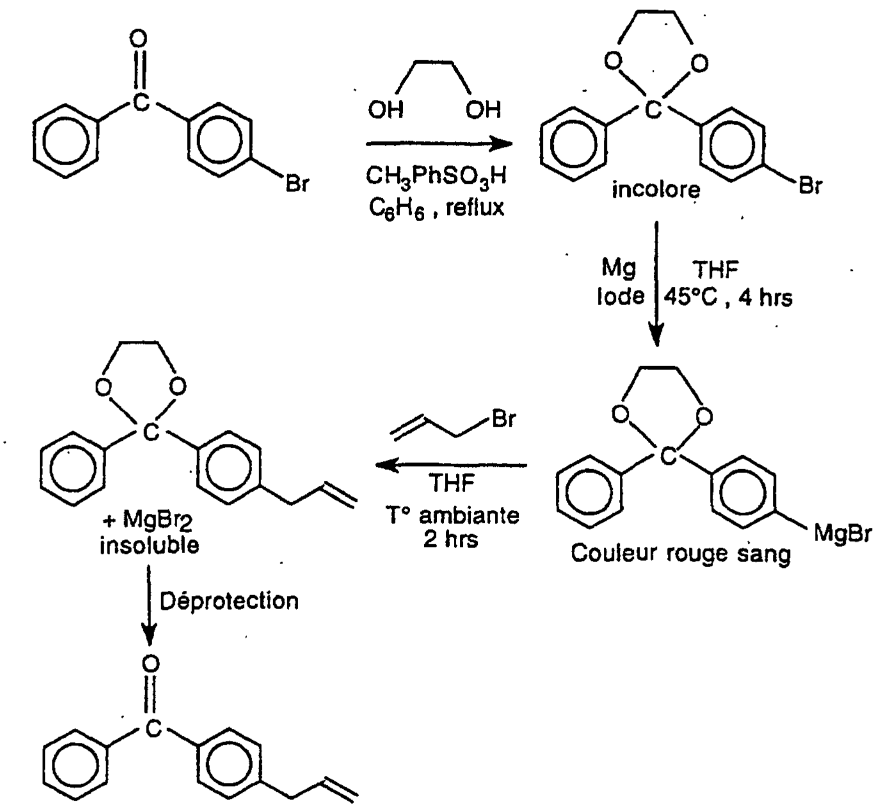


1) Protection de la fonction carbonyle par synthèse d'un acétal

La première étape consiste à protéger la fonction carbonyle de la bromobenzophénone par la formation d'un acétal cyclique. Expérimentalement, cette réaction est réalisée en solution dans le benzène en utilisant un appareil de Dean Starck pour éliminer l'eau libérée dans le procédé.

Les conditions opératoires sont les suivantes :

Dans un ballon de 250 ml, surmonté d'un Dean Starck et d'un condenseur, on introduit :

| | | |
|---|---|---|
| 4-bromobenzophénone | 80,0 g | soit 0,306 mole |
| Ethylèneglycol | 18,8 ml | soit 0,337 mole (10% en excès) |
| Benzène | 130 ml | |
| Acide p-toluène sulfonique | 100 mg | |

On chauffe le mélange réactionnel à 110°C au moyen d'un bain d'huile. La quantité théorique d'eau libérée dans le procédé dans le cas d'un rendement quantitatif est de 5,15 ml. La réaction est poursuivie pendant 56 heures. Le mélange réactionnel initialement trouble est limpide et présente une légère coloration jaune. 9,478 g d'eau ont été recueillis en totalité alors que la réaction n'en libère que 5,15 g. La différence est attribuée à la présence d'eau dans le benzène brut utilisé. Le solvant est ensuite évaporé à 60°C au moyen d'un évaporateur rotatif. Des micro gouttes d'eau ou d'éthylèneglycol résiduel semblent être présentes dans le milieu.

On réalisé une dévolatilisation complémentaire en utilisant un bain d'huile thermostaté à 50°C couplé au vide d'une pompe à palettes.

Finalement, on a confirmé la structure du composé obtenu par RMN $^{1}H$ et $^{13}C$ dans $CDCl_3$.

Il subsiste cependant une faible quantité de benzophénone non protégée qu'il est nécessaire de séparer. En effet, ce composé conduirait à la formation de produits indésirables par couplage avec l'organomagnésien qui sera préparé lors de l'étape suivante.

A ce stade de la synthèse, le produit est une huile visqueuse légèrement jaunâtre qui commence à cristalliser.

Après une journée au repos, le produit a pris en masse dans sa totalité. On ajoute alors 100 ml d'éthanol et on y dissout le produit à une température voisine de 50°C. Aucune cristallisation n'est induite lors du refroidissement de la solution. On ajoute alors quelques cristaux de 4-bromobenzophénone et on met le mélange dans un réfrigérateur pour une trentaine de minutes. Ce traitement permet d'obtenir de beaux cristaux blancs en forme de paillettes. La recristallisation est ensuite poursuivie selon les techniques usuelles et la pureté du composé ainsi obtenu est vérifiée par RMN.

Les échantillons recueillis en fin de recristallisation sont analysés par RMN $^1H$ afin d'en vérifier la pureté, avant de les mélanger aux fractions de tête. Il s'avère que pour les deux dernières fractions recueillies, cette pureté n'est plus satisfaisante.

La masse totale d'acétal pur recueilli après recristallisation est de 70,0 g.

Le rendement de synthèse, recristallisation comprise, calculé par rapport à la quantité initiale de 4-bromobenzophénone engagée est donc de 74,9%.

**2) <u>Synthèse de l'organomagnésien</u>**

Cette deuxième étape consiste à faire réagir l'acétal de la 4-bromobenzophénone avec du magnésium en tournures, afin de préparer le dérivé intermédiaire organomagnésien qui servira ultérieurement lors de la réaction de couplage avec le bromure d'allyle.

Les conditions opératoires sont les suivantes :

La réaction est réalisée sous léger flux d'argon dans un ballon tricol de 250 ml, équipé d'un réfrigérant à boules surmonté d'un tube à $CaCl_2$, d'une ampoule à addition de 100 ml, et d'un raccord en Y muni d'un thermomètre et d'une entrée d'argon.

La réaction est exothermique, cependant lorsqu'un chauffage d'appoint sera nécessaire, ce dernier sera réalisé par bain d'eau thermostaté à 45°C.

| | | |
|---|---|---|
| Acétal de la 4-BrPh | 20,097 g | dissout dans 40 ml de THF anhydre (0,0686 mole) |
| Mg en tournures | 1,71 g | dans 10 ml de THF anhydre (0,0704 mole) |
| Iode | un cristal | |

Dans un premier temps, le réacteur est purgé par de l'argon pendant environ 15 minutes. On introduit le magnésium, 10 ml de THF anhydre et un cristal d'iode. La solution d'acétal est préparée dans un erlenmeyer, transvasée dans l'ampoule à addition, puis cette dernière est montée sur le réacteur. On purge à nouveau le réacteur à l'argon pendant quelques minutes. Environ 5 ml de la solution d'acétal sont ensuite coulés dans le réacteur, puis sans agiter, ce mélange est ensuite chauffé à l'aide d'un sèche cheveux jusqu'à l'apparition de bulles à la surface des tournures de magnésium, on constate un léger trouble dans le milieu réactionnel, puis l'apparition rapide de la couleur rouge sang de l'organomagnésien formé. L'agitation est alors mise en route et la solution d'acétal additionnée au goutte à goutte.

La réaction étant exothermique, la vitesse d'addition est ajustée de manière à maintenir la température du milieu réactionnel proche de 45°C. Si nécessaire, le bain d'eau thermostaté pourra être utilisé.

La durée totale de l'addition est de 90 minutes, puis la réaction est encore poursuivie dans ces conditions durant 90 minutes supplémentaires. En fin de réaction, il subsiste encore une faible quantité de tournures de magnésium non consommé dans le milieu et la solution ainsi obtenue est de couleur rouge sang.

**3) <u>Réaction de couplage</u>**

Avant de procéder à la réaction de couplage avec le bromure d'allyle, on laisse refroidir le mélange réactionnel jusqu'à température ambiante.

Les conditions opératoires sont les suivantes :

On prépare une solution de bromure d'allyle dans le THF dans une ampoule à addition de 100 ml par dissolution de 5,8 ml du bromure (8,108 g, soit 0,0671 mole) dans 25 ml de THF anhydre. On monte ensuite l'ampoule sur le réacteur et on commence l'addition au goutte à goutte.

On constate très rapidement l'apparition d'un composé insoluble dans le milieu réactionnel. Ce dernier est en principe le bromure de magnésium qui est libéré lors de l'avancement de la réaction. La durée totale de l'addition est d'une heure, puis l'agitation est encore poursuivie dans les mêmes conditions pendant une heure supplémentaire.

En fin de réaction, on dilue le mélange réactionnel de couleur orangé avec un peu de THF puis on filtre sur un verre fritté afin d'en séparer le magnésium résiduel ainsi que le sel précipité formé durant la synthèse. On évapore ensuite le THF au moyen d'un évaporateur rotatif. Le résidu ainsi obtenu est huileux et contient une proportion non négligeable d'un composé insoluble, probablement un résidu de bromure de magnésium. On reprend le résidu dans

250 ml de dichlorométhane (solution limpide jaune orangé) puis on extrait successivement à trois reprises avec 50 ml d'eau distillée.

Lors de la première extraction, la séparation de phase n'est pas nette, mais est constituée d'une émulsion très difficile à casser. Une quantité importante du composé synthétisé est très probablement prisonnière de cette émulsion et doit être récupérée. Les phases aqueuses et émulsionnées sont donc réextraites avec un peu de dichlorométhane.

Finalement, on filtre la phase organique sur un verre fritté de porosité 4, puis on sèche sur du sulfate de magnésium avant d'éliminer le dichlorométhane. Le résidu orangé isolé est caractérisé par RMN.

On obtient ainsi 15,25 g de produit brut.

**4) Réaction de déprotection**

Plusieurs voies de déprotection du carbonyle sont possibles. Citons l'hydrolyse acide et l'échange de dioxolane par catalyse acide. C'est cette dernière voie qui sera retenue ici.

On a effectué la réaction de déprotection sur la totalité du produit recueilli lors des deux synthèses effectuées. (La deuxième synthèse est décrite ci-après).

| | |
|---|---|
| Acétal fonctionalisé | 29,92 g |
| Acétone | 150 ml |
| $H_2SO_4$ 95% | 0,05 ml |

La température de réaction est fixée par la température d'ébullition de l'acétone. La durée totale de la réaction est de 2 heures. La solution initialement jaune-orangé vire au rouge. Le solvant est évaporé au moyen d'un évaporateur rotatif, puis on réalise une dévolatilisation complémentaire à l'aide d'une pompe à palettes à une température proche de 45°C. On recueille ainsi 26,92 g d'une huile opaque de couleur brune.

On a séparé une faible quantité de produit cristallisé en utilisant un mélange constitué de 450 ml d'éthanol et de 50 ml de benzène. Après filtration et caractérisation des cristaux par RMN $^1$H, il s'est avéré qu'il s'agissait là de 4-bromobenzophénone. On concentre alors le filtrat jusqu'à ce qu'il ne subsiste qu'environ 50 à 100 ml de solution, puis on le refroidit à 0°C. Une pâte se dépose au fond du ballon. On répète le procédé de cristallisation, mais peu de cristaux se déposent. Il reste 24 g d'huile.

On distille 23,24 g d'huile dans le four à boules sous un vide de $10^{-2}$ mmHg dans une plage de températures s'étalant de 175 à 250°C. On recueille 11,94 g d'une huile légèrement jaune constituée de deux phases. La phase minoritaire est attribuée à de l'éthylèneglycol qui résulterait d'une déprotection thermique catalysée par l'acide sulfurique, d'un résidu d'acétal présent dans l'huile.

On centrifuge le distillat pour en séparer l'éthylèneglycol (30 minutes à 10.000 t/min.), puis on redistille à 185°C sous ($10^{-2}$ mmHg) 1,33Pa. Une faible quantité d'éthylèneglycol est à nouveau libérée et le distillat est légèrement jaune.

Les trois fractions (tête, milieu, queue) sont caractérisées par RMN, puis les fractions de tête et milieu sont réunies (11,02+0,58=11,60 g).

En raison de la présence de l'éthylèneglycol, on reprend le tout dans 100 ml de benzène, on rajoute trois spatules de gel de silice et l'on agite dans le but de favoriser l'adsorption de l'éthylèneglycol sur la silice. Après filtration sur verre fritté et évaporation du solvant, une analyse par RMN $^1$H montre que la quantité d'éthylèneglycol présent a été réduite de moitié.

**2ème synthèse de l'allyl-4 benzophénone**

L'appareillage et les conditions opératoires sont similaires.

**Synthèse du dérivé organomagnésien**

Dans l'ampoule à addition, on place :
Acétal de la bromobenzophénone : 20,0 g (0,0683 mole) dans 75 ml de THF anhydre.

Dans le ballon, on place :

| | |
|---|---|
| Mg en tournures | 1,63 g (0,0671 mole) |
| THF anhydre | 15 ml |

(suite)

| | |
|---|---|
| un cristal d'iode | |

On additionne 5 à 10 ml de la solution d'acétal, puis sans agiter, on chauffe ce mélange jusqu'à l'apparition d'un dégagement gazeux à la surface du métal. L'agitation est alors mise en route et l'acétal additionné au goutte à goutte lentement. La coloration rouge sang du dérivé organomagnésien se développe quasi instantanément dans le milieu réactionnel.

On utilise un bain d'eau thermostaté comme chauffage d'appoint et on règle la vitesse d'addition de manière à maintenir la température entre 43 et 47°C.

La durée totale de l'addition est de 2 heures, au bout desquelles il reste encore un résidu de magnésium non consommé. On poursuit la réaction dans les mêmes conditions pendant 2 heures supplémentaires.

**Réaction de couplage avec le bromure d'allyle :**

On prépare une solution de bromure d'allyle par dilution de 6,0 ml de bromure d'allyle (0,0692 mole) dans 50 ml de THF anhydre.

Après refroidissement de la solution d'organomagnésien à température ambiante, on ajoute lentement goutte à goutte la solution de bromure d'allyle en environ 2 heures. Un précipité apparaît rapidement dans le milieu réactionnel.

En fin de réaction, on dilue cette solution avec un peu de THF, puis on filtre sur verre fritté de porosité 4, afin d'en séparer le sel insoluble ainsi que le magnésium résiduel. Le filtrat est ensuite évaporé au moyen d'un évaporateur rotatif à 50°C et on isole un résidu pâteux de couleur orangé qui est repris dans environ 250 ml de dichlorométhane, puis extrait à plusieurs reprises à l'eau distillée. Une fois de plus, on observe la formation d'une phase émulsionnée très stable lors de la première extraction. On sèche ensuite la phase organique de couleur jaune orangé sur sulfate de magnésium, puis on évapore le solvant.

On recueille 15,65 g d'une huile de couleur orange.

Une RMN $^1H$ de contrôle confirme la nature du composé ainsi préparé, ainsi que la présence d'impuretés qu'il faudra éliminer après l'étape de déprotection de la fonction carbonyle.

Préparation de l'allyloxyméthyl-4 benzophénol

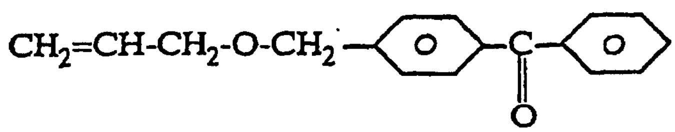

On dissout 5 g de bromométhylbenzophénone dans 20 ml de THF anhydre et on place la solution obtenue dans une burette de Schlank.

Dans un ballon de 500 ml muni d'un thermomètre, d'une burette à arnorceur, d'une burette d'introduction d'alcool allylique et de la burette de Schlank, on introduit 40 ml de THF. On neutralise les impuretés du solvant avec 2 à 3 gouttes de naphtalène-potassium

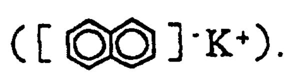

Puis on introduit 1,2 ml ($1,82.10^{-2}$ mole) d'alcool allylique distillé. On ajoute alors 20,2 ml de naphtalène-potassium. Il se forme un précipité blanc au cours de l'addition. On ajoute le contenu de la burette de Schlank. La température augmente de 5 à 10°C. Le précipité augmente d'importance et devient rouge-brun. On laisse sous agitation pendant 3 heures. La couleur du milieu réactionnel s'éclaircit et la température diminue. On chasse alors le THF et on reprend avec du benzène. Un précipité important subsiste. On lave avec de l'eau et on filtre la phase organique. On sèche, puis on élimine le benzène.

On recueille 5,69 g d'un liquide jaune foncé. On place le liquide obtenu dans un sublimateur pendant 5 heures à 100°C. Le résidu liquide obtenu représente 3,40 g (74%). On distille 1,7 g de ce résidu dans un four à boules et on

recueille 0,7 g (57%) de produit passant à 175°C sous ($10^{-2}$mmHg) 1,33Pa.

On distille les 1,7 g restant dans les mêmes conditions et on recueille 0,61 g (41%) de produit.

Le distillat comprend une partie solide à la température ambiante. On filtre le distillat sur filtre Millipore® (0,5 µm) et on recueille finalement 1,05 g (23%) du produit attendu répondant à la formule :

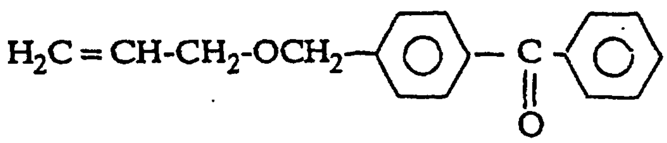

Préparation de l'hydroxy-2 phényl-1 méthyl-2 hexène-5 one-1.

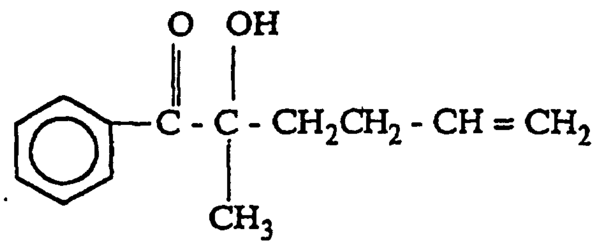

(Va)

**a) Schéma de synthèse.**

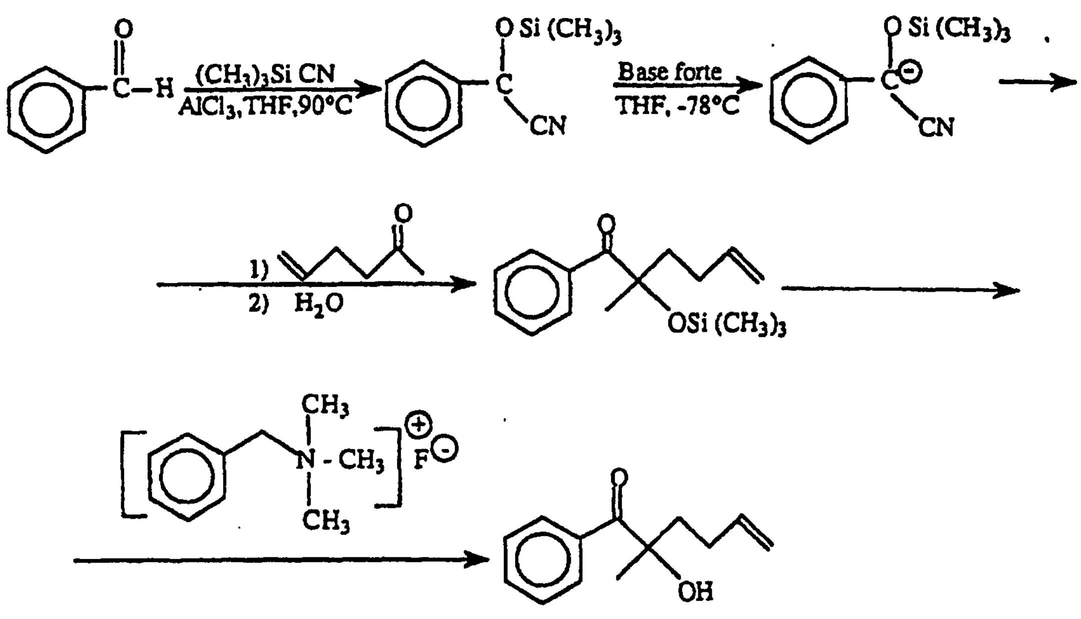


**b) Synthèse du phényl triméthylsiloxyacétonitrile.**

5 ml (3,72 g; 37,5 mmoles) de triméthylsilyl cyanure (prélevé à l'aide d'une seringue sèche) sont additionnés à une solution de 3,98 g (37,5 mmoles) de benzaldéhyde fraîchement distillé et 20 mg d'$AlCl_3$ dans du THF.

Le montage est mis sous atmosphère inerte d'argon et le mélange est chauffé sous reflux à 90°C. Après 10 heures d'agitation, le solvant est évaporé et le résidu distillé à 80°C 13,3 Pa (0,1 mmHg) au four à boules.
On obtient le produit attendu avec un rendement de 95%.
On a confirmé la structure du produit obtenu par spectrométries IR (spectromètre Perkin Elmer) et RMN (AC 200 MHz).

**c) Synthèse de l'hydroxy-2 phényl-1 méthyl-2 hexène-5 one-1.**

Une solution de tert-butyl-lithium dans l'hexane (tert-BuLi/hexane) (1,6M; 20 ml; 31,8 mmoles) est ajoutée, sous atmosphère d'argon, lentement à une solution de di-isopropylamine (3,23 g; 31,9 mmoles) dans THF (20 ml) à -78°C.
Le mélange est maintenu à cette température pendant 45 minutes. On ajoute alors goutte à goutte, une solution de phényl triméthylsiloxyacétonitrile (6,54 g; 31,8 mmoles) dans THF (50 ml).
L'agitation est maintenue pendant 45 minutes, puis on ajoute lentement à la solution brune intense une solution d'hexène-5 one-2 (3,26 g; 31,8 mmoles) dans THF.
Le mélange est maintenu sous agitation. Après 2 heures à -78°C, on retire le bain de glace. Lorsque la température du ballon atteint 0°C, on ajoute 300 ml d'eau. La solution obtenue est introduite dans une ampoule à décanter et extraite avec 400 ml de $CH_2Cl_2$ après plusieurs agitations.
La solution organique récupérée est concentrée.
Déprotection du groupement alcool : A la solution organique récupérée, on ajoute 5,4 g de fluorure de benzyltriméthylammonium, 20 ml de THF et 1 ml d'$H_2O$. Après 4 heures d'agitation, la solution est lavée avec 4x300 ml d'eau puis extraite à l'éther. La solution éthérée est concentrée et distillée au four à boules (115°C à (0,1 mmHg) 13,3Pa)
On obtient le produit attendu avec un rendement de 35%.
La structure du produit a été confirmée par spectrométries IR et RMN.

Préparation de (o-allylphényl)-1 triméthylsiloxy-2 méthyl-2 butanone-1.

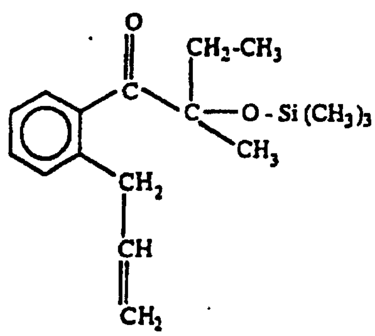

(Vf)

a) Schéma de synthèse

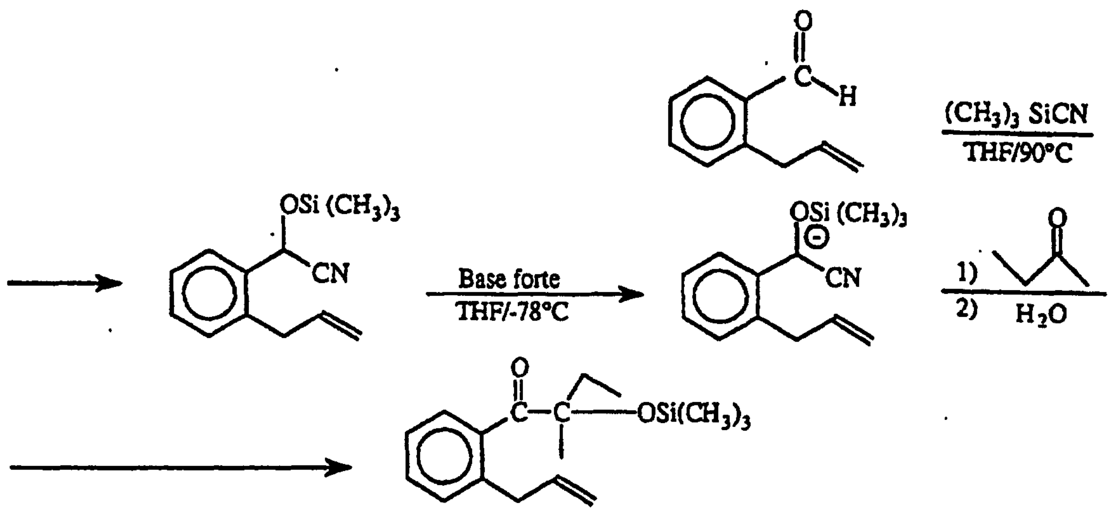


**b) Synthèse de o-allyl phényl triméthylsiloxyacétonitrile.**

0,8 ml (600 mg; 6,04 mmoles) de triméthylsilylcyanure est additionné à une solution de 883 mg d'allyl benzaldéhyde distillé et 5 mg d'$AlCl_3$ anhydre dans du THF.
Le montage est mis sous atmosphère inerte d'argon et le mélange est chauffé sous reflux à 90°C. Après 10 heures d'agitation, le solvant est évaporé et le résidu distillé à 130°C, 13.3 Pa (0,1 mm Hg) au four à boules.
On obtient le produit attendu avec un rendement de 85%.
La structure du produit a été confirmée par spectrométries IR et RMN.

**c) Synthèse de (o-allyl phényl)-1 triméthylsiloxy-2 méthyl-2 butanone-1.**

Une solution de tert-BuLi/hexane (1,6M; 2,38 ml; 3,81 mmoles) est ajoutée sous atmosphère d'argon lentement à une solution de di-isopropylamine (395 mg; 3,9 mmoles) dans THF (20 ml) à -78°C.
Le mélange est maintenu à cette température pendant 15 minutes. On ajoute alors goutte à goutte, une solution d'o-allyl phényl triméthyl siloxyacétonitrile (938 mg; 3,81 mmoles) dans THF (50 ml).
L'agitation est maintenue pendant 10 minutes, puis on ajoute lentement à la solution brune intense une solution de butanone (274 mg; 3,81 mmoles) dans THF.
Le mélange est maintenu sous agitation. Après 3 heures à -78°C, on retire le bain de glace. Lorsque la température du ballon atteint 0°C, on ajoute 100 ml d'eau. La solution obtenue est introduite dans une ampoule à décanter et extraite avec 400 ml de $CH_2Cl_2$ après plusieurs agitations.
La solution organique récupérée est concentrée puis distillée à 125°C, 13,3 Pa (0,1 mmHg) au four à boules.
On obtient le produit attendu avec un rendement de 35%. La structure du produit a été confirmée par spectrométries IR et RMN.

Préparation de (o-allyl phényl)-1 triméthylsiloxy-2 méthyl-2 hexène-5 one-1.

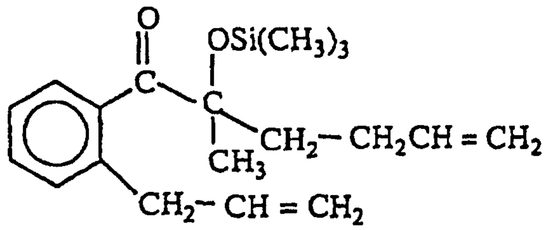

(Vg)

**a) Schéma de synthèse**

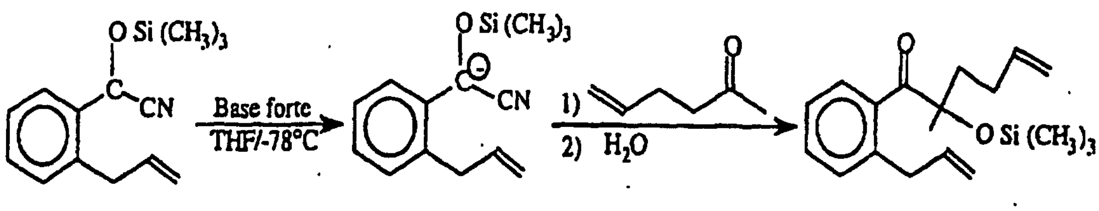


**b) Synthèse de (o-allyl phényl)-1 triméthylsiloxy-2 méthyl-2 hexène-5 one-1.**

Une solution de tert-BuLi/hexane (1,5M, 3,1 ml, 4,7 mmoles) est ajoutée lentement sous atmosphère d'argon à une solution de di-isopropylamine (486 mg; 4,8 mmoles) dans THF (50 ml) à -78°C.
Le mélange est maintenu à cette température pendant 15 minutes. On ajoute alors goutte à goutte, une solution d'o-allyl phényl triméthyl siloxyacétonitrile (1,152 g; 4,7 mmoles) dans THF (50 ml).
L'agitation est maintenue pendant 5 minutes, puis on ajoute lentement à la solution noire intense une solution d'hexène-5 one-2 (471 mg, 4,8 mmoles) dans THF.
Le mélange est maintenu sous agitation. Après 3 heures à -78°C, on retire le bain de glace. Lorsque la température du ballon atteint 0°C, on ajoute 400 ml d'eau. La solution obtenue est introduite dans une ampoule à décanter et extraite avec 400 ml de $CH_2Cl_2$ après plusieurs agitations.
La solution organique récupérée est concentrée puis distillée à 150°C, 13.3 Pa (0,1 mmHg) au four à boules.
On obtient le produit attendu avec un rendement de 45%.
La structure du produit a été confirmée par spectrométries IR et RMN.

Préparation de la triméthylsiloxy-2 phényl-1(3-butène)-2 hexène-5 one-1.

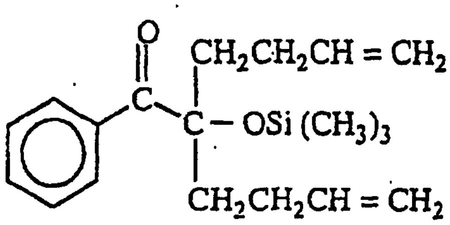

(Vb)

**a) Schéma de synthèse.**

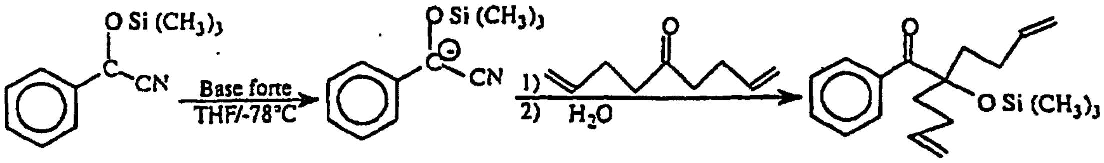


**b) Synthèse du 1,8-nonadiène one-5.**

Une solution de tert-BuLi/hexane (1,55M; 13 ml; 20 mmoles) est ajoutée lentement sous atmosphère d'argon à une solution de di-isopropylamine (2,12 g; 21 mmoles) dans THF (20 ml) à -78°C.
Le mélange est maintenu à cette température pendant 45 minutes. On ajoute alors goutte à goutte, une solution d'hexène-5 one-2 (1,96 g; 20 mmoles) dans THF (20 ml).
L'agitation est maintenue pendant 60 minutes, puis on ajoute lentement à la solution incolore 1,8 ml de bromure d'allyle. L'agitation est maintenue pendant 12 heures à température ambiante.
La solution rouge résultante est filtrée sur 2 cm de silice. On obtient alors une solution jaune que l'on concentre. Le résidu est distillé au four à boules (50°C; (2 mmHg) 267 Pa)
On obtient le produit attendu avec un rendement de 65%.
On a confirmé la structure par spectrométries IR et RMN.

**c) Synthèse de la triméthylsiloay-2 phényl-1 (3-butène)-2 hexène-5 one-1.**

Une solution de tert-BuLi/hexane (1,55M, 4,1 ml, 6,3 mmoles) est lentement ajoutée sous atmosphère d'argon à une solution de di-isopropylamine (648 g; 6,4 mmoles) dans THF (20 ml) à -78°C.
Le mélange est maintenu à cette température pendant 15 minutes. On ajoute alors goutte à goutte, une solution de phényl triméthylsiloxyacétonitrile (1,29 g; 6,3 mmoles) dans THF (50 ml).
L'agitation est maintenue pendant 20 minutes, puis on ajoute lentement à la solution brune intense une solution de nonadiéne-1,8 one-5 (867 mg, 6,3 mmoles) dans THF.
Le mélange est maintenu sous agitation. Après 3 heures à -78°C, on retire le bain de glace.
Lorsque la température du ballon atteint 0°C, on ajoute 200 ml d'eau. La solution obtenue est introduite dans une ampoule à décanter et extraite avec 400 ml de $CH_2Cl_2$ après plusieurs agitations.
La solution organique récupérée est concentrée puis distillée à 150°C, 13,3 Pa (0,1 mmHg) au four à boules.
On obtient le produit attendu avec un rendement de 30%.
La structure a été confirmée par spectrométries IR et RMN.

<u>Préparation de l'ortho-di[(o-triméthylsiloxy)-2 (3-butène)-2 hexène-5 one-1]phényl-1.</u>

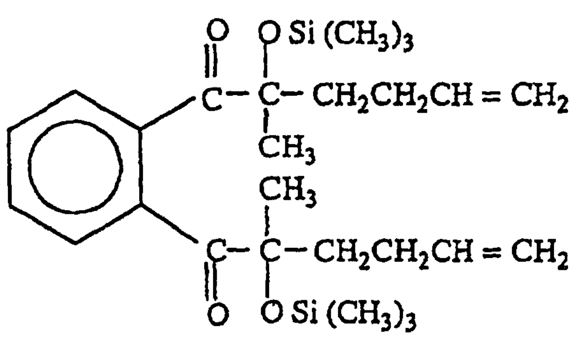

(Vd)

**a) Schéma de synthèse**

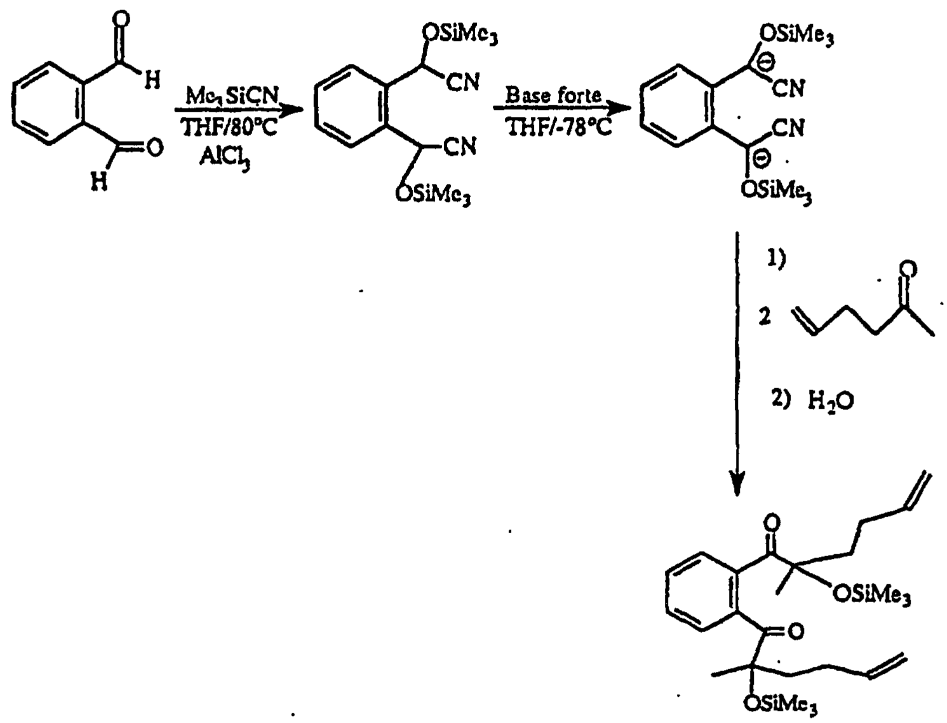


**b) Synthèse de l'ortho-phtalique di(triméthylsiloxyacétonitrile).**

3,565 g (36 mmoles) de triméthylsilylcyanure liquide (prélevée à l'aide d'une seringue sèche) est additionnée à une solution de 2,41 g (36 mmoles) de l'ortho-phtaldicarboxyaldéhyde et 20 ml d'$AlCl_3$ dans du THF.
Le montage est mis sous atmosphère inerte d'argon et le mélange est chauffé sous reflux à 90°C. Après 10 heures d'agitation, le solvant est évaporé et le résidu distillé à 175°C 13,3 Pa (0,1 mmHg) au four à boules.
On obtient le produit attendu avec un rendement de 75%.
On a confirmé la structure du produit par spectrométries IR et RMN.

c) La suite de la synthèse du composé (Vd) s'effectue à partir de l'ortho-phtal di(triméthylsiloxyacétonitrile) comme précédemment.

<u>Préparation d'un macrophotoamorceur du type vinylsilane Irgacure® polydiméthylsiloxane-vinylsilane Irgacure®</u>

On met dans un ballon de 100 ml 0,61 g ($8{,}1.10^{-4}$ mole) d'un PDMS à terminaisons silyles ayant une masse molaire moyenne en nombre $M_n$ d'environ 750 et 0,5 g de vinylsilane Irgacure® de formule :

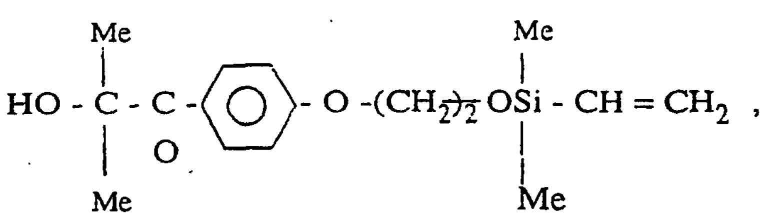

préalablement dissous dans 5 ml de cyclohexane (qualité anionique). On ajoute 0,12 ml ($6{,}646.10^{-3}$ mole) d'une solution de $H_2PtCl_6$, $6H_2O$ dans 50 ml d'isopropanol et on chauffe 8 heures à 70°C. On laisse reposer une nuit puis on filtre

sur filtre Millipore® (0,45 µm).

On obtient ainsi le produit attendu répondant à la formule :

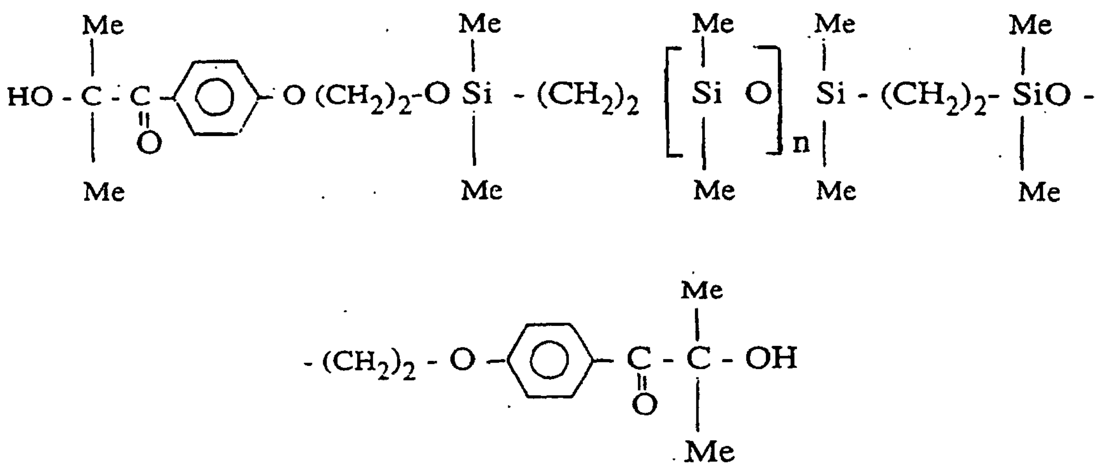

Préparation d'un macrophotoamorceur difonctionnel composé (Va) - Polydiméthylsiloxane - composé (Va).

**a) Schéma de synthèse**

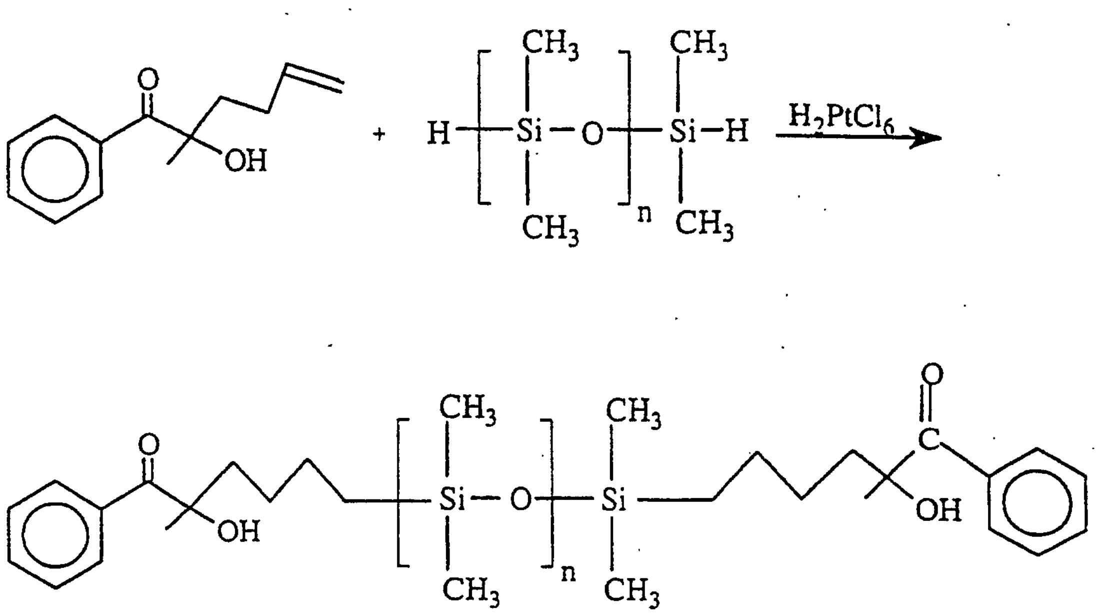


**b) Mode opératoire**

Dans un tricol de 250 ml, on ajoute successivement 1,53 g (4,08 mmoles) d'α,ω-dihydropolydiméthyl siloxane (PDMS), puis 833 mg (8,16 mmoles) d'hydroxy-2 phényl-1 méthyl-2 hexène-5 one-1. Après trois vide/argon, on introduit 50 ml de toluène anhydre puis 2 ml d'une solution de $H_2PtCl_6$ dans l'isopropanol (6,646 mmoles), puis on chauffe à 80°C.

Après 24 heures d'agitation, le solvant est évaporé. Le résidu visqueux obtenu est précipité dans 100 ml de MeOH et séché. Afin d'éliminer les traces de catalyseur, le filtrat (MeOH) est évaporé et filtré sur 2 cm de $SiO_2$ (traitée avec une solution de cyclohexane à 5% de triéthylamine, éluant $CH_2Cl_2$), puis séché sous vide.

Préparation d'un macrophotoamorceur difonctionnel composé (Ve) - polydiméthylsiloxane - composé (Ve)

**a) Schéma de synthèse.**

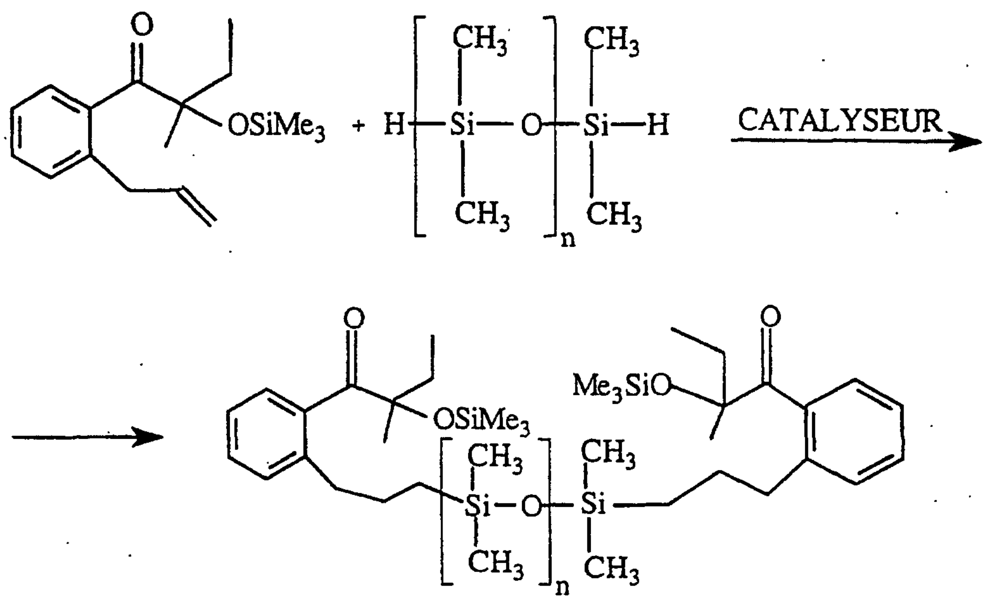


Dans un tricol de 250 ml, on ajoute successivement 397 mg (0,53 mmole) d'α,ω-dihydropolydiméthyl siloxane (PDMS), puis 307 mg (1,06 mmole) d'(o-allylphényl)-1 triméthylsiloxy 2-méthyl-2 butanone-1. Après trois vide/argon, on introduit 50 ml de toluène anhydre, puis 1,6 ml d'une solution de platine divinyl tétraméthyl disiloxane dans le toluène (0,392 mmole).

Après 24 heures d'agitation à température ambiante, le solvant est évaporé. Le résidu visqueux obtenu est précipité dans 150 ml de méthanol et séché. Afin d'éliminer les traces de catalyseur, le filtrat (MeOH) est évaporé et filtré sur 2 cm de $SiO_2$ (traitée avec une solution de cyclohexane à 5% de triéthylamine, éluant $CH_2Cl_2$), puis séché sous vide.

Préparation de macrophotoamorceurs multifonctionnels.

**I.** A partir de triméthylsiloxy-2 phényl-1 (3-butène)-2 hexène-5 one-1.

**a) Schéma de synthèse**

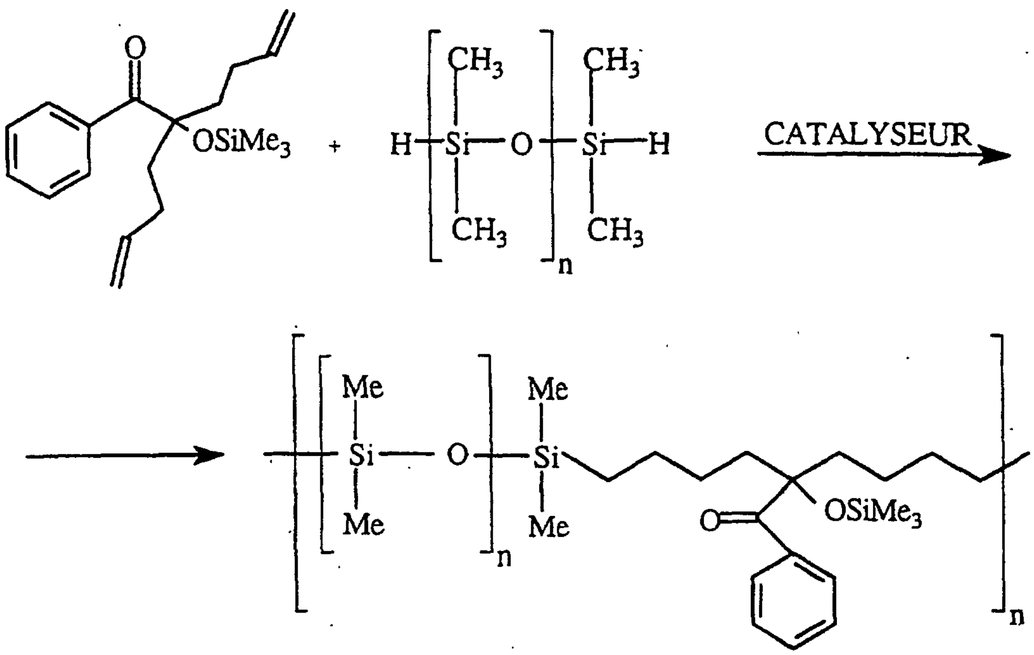


**b) Mode opératoire**

Dans un tricol de 250 ml, on ajoute successivement 681 mg (0,9 mmole) d'$\alpha,\omega$-dihydropolydiméthylsiloxane (PDMS), puis 287 mg (0,9 mmole) de triméthylsiloxy-2 phényl-1 (3-butène)-2 hexène-5 one-1.

Après trois vide/argon, on introduit 50 ml de toluène anhydre puis 2,73 ml d'une solution de platine divinyl tétraméthyl disiloxane dans le toluène (0,392 mmole/l).

Après 48 heures d'agitation à température ambiante, le solvant est évaporé. Le résidu visqueux obtenu est précipité dans 100 ml de méthanol. Après 3 jours de décantation, on retire le méthanol et l'on sèche le précipité. La phase organique (MeOH) est évaporée et filtrée sur 2 cm de $SiO_2$ (traitée avec une solution de cyclohexane à 5% de triéthylamine, éluant $CH_2Cl_2$), puis séchée sous vide.

**II**. A partir de (o-allyl phényl)-1 triméthylsiloxy-2 méthyl-2 hexène-5 one-1.

Dans un tricol de 250 ml, on ajoute successivement 1,8 g (2,4 mmoles) de $\alpha,\omega$-dihydropolydiméthylsiloxane (PDMS), puis 760 mg (2,4 mmoles) de (o-allyl phényl)-1 triméthylsiloxy-2 méthyl-2 hexène-5 one-1. Après trois vide/argon, on introduit 50 ml de toluène anhydre puis 5 ml d'une solution de platine divinyl tétraméthyl disiloxane dans le toluène (0,392 mmole/l).

Après 48 heures d'agitation à température ambiante, le solvant est évaporé. Le résidu visqueux obtenu est précipité dans 100 ml de méthanol. Après 4 jours de décantation, on retire le méthanol et l'on sèche le précipité. La phase organique (MeOH) est évaporée et filtrée sur 2 cm de $SiO_2$ (traitée avec une solution de cyclohexane à 5% de triéthylamine, éluant $CH_2Cl_2$), puis séchée sous vide.

**Synthèse d'un macrophotoamorceur de formule :**

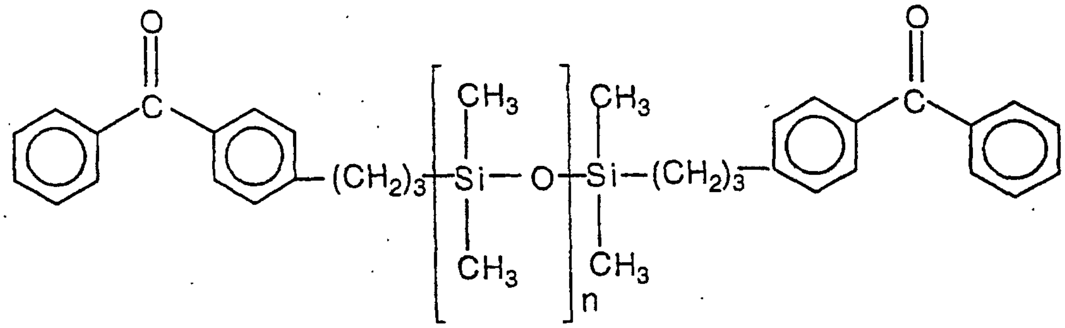

(ALBP - PDMS - ALBP).

Dans un ballon tricol de 250 ml équipé d'un réfrigérant à boules, on pèse directement 5,06 g ($6{,}75.10^{-3}$ mole) d'huile polydiméthylsiloxane à terminaisons silyles Mn = 750, ainsi que 3,0 g ($1{,}35.10^{-2}$ mole) d'allyl-4 benzophénone. Après trois purges successives à l'argon, on introduit 50 ml de benzène anhydre et 1,023 ml d'une solution de catalyseur ($H_2PtCl_6$, 6 $H_2O$ - solution de $6{,}646.10^{-3}$ M dans l'isopropanol). On chauffe ensuite le mélange à 70°C durant 8 heures.

En fin de réaction, la solution est limpide. Le solvant est évaporé et l'on observe alors la présence de quelques particules solides (catalyseur) qui sont éliminées par filtration sur filtre Millipore® de 0,5 µm. On recueille 8,01 g du produit attendu.

**Synthèses d'un co-amorceur fonctionnalisé et d'un coamorceur à longue chaîne**

**A. Synthèse de la 4-diméthylvinylsilane-N,N-diméthylaniline :**

1.

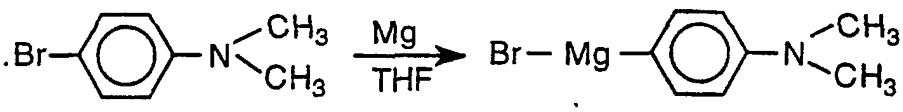


2.

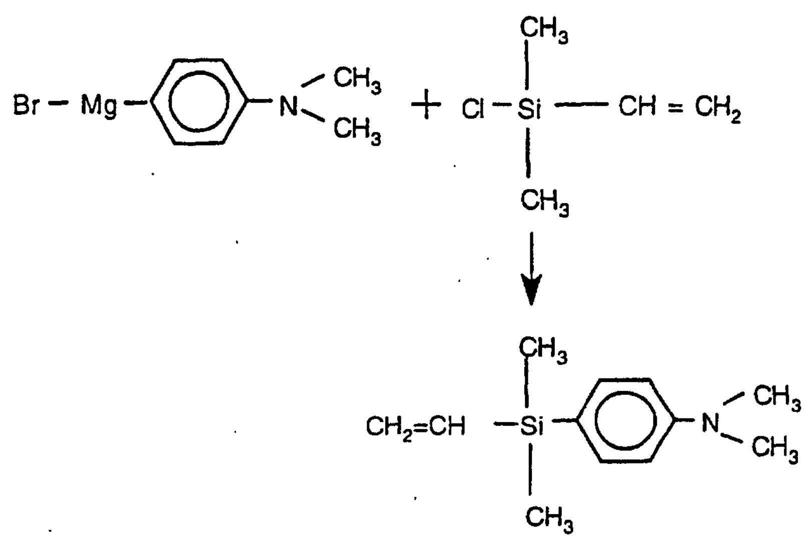

(A)

Mode opératoire :

Tous les produits doivent être purifiés avant de commencer la synthèse. Le THF est distillé sous argon, sur fil de sodium, à pression atmosphérique. La 4-bromo-N,N-diméthylaniline (Aldrich) est conditionnée sous argon. Le chlorodiméthylvinylsilane (Aldrich) est distillé à pression atmosphérique, sous un léger flux d'argon (Teb.=81°C). Ensuite, on prépare dans une ampoule sous argon, une solution de THF anhydre (66 ml, 2/3 du volume total de THF dans le milieu réactionnel) et de 4-bromo-N,N-diméthylvinylaniline (m = 20,01 g, n = 0,1 mole).

Dans un réacteur muni d'une agitation mécanique, d'un réfrigérant et d'un thermomètre, on introduit 2,673 g (0,11 mole) de magnésium, ainsi qu'un cristal d'iode. Le montage est alors conditionné sous argon. Sous un flux d'argon, on introduit 30 ml de THF. La solution devient alors jaune-brun. 1/10 au maximum de la solution brome/THF est alors ajoutée sous argon. Après 1/2 heure de réaction, la solution devient trouble-incolore, puis violette après 3/4 d'heure. On observe alors un léger échauffement et il se forme un reflux.

On ajoute ensuite goutte à goutte la solution bromée, sous argon. A la fin de l'ajout, on chauffe le milieu réactionnel à 50°C pendant deux heures. On laisse la température de la solution revenir à l'ambiante.

On ajoute ensuite 13,81 ml (0,1 mole) de chlorure de silane goutte à goutte. On observe un échauffement du milieu réactionnel. A la fin de l'ajout, on poursuit l'agitation pendant 15 minutes, puis on ajoute 8 ml d'eau. Une émulsion blanche, ainsi qu'un reflux, se produisent. Après avoir laissé la température du milieu réactionnel revenir à l'ambiante, on ajoute 100 ml d'$H_2O$, et 100 ml d'éther. La solution est filtrée. La phase organique est séparée de la phase aqueuse, lavée avec une solution saturée de $NaCO_3$. La solution de couleur jaune est séchée sur $MgSO_4$, les solvants sont chassés.

Rendement brut = 91%
Masse brute = 18,7 g.

Le produit brut est distillé sous vide, $T_{0,1}$ eb. = 79°C, rendement = 50%.

**B. Condensation de la 4-diméthylvinylsilane-N,N-diméthvlaniline sur un PDMS.**

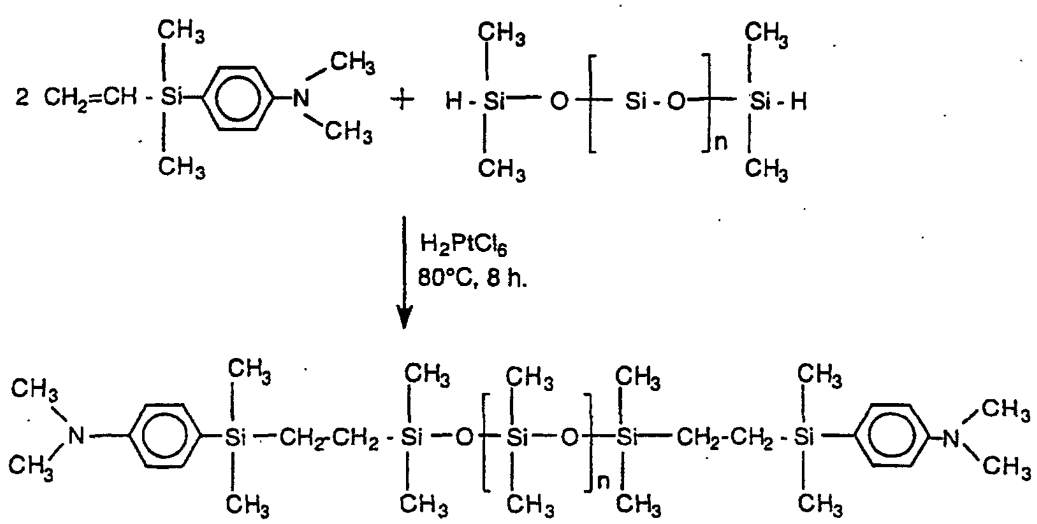


Mode opératoire :

Dans un tricol conditionné sous argon, muni d'une agitation magnétique, on introduit 1 g (0,0049 mole) du produit obtenu à l'étape précédente, puis 1,8293 g (0,0024 mole) de PDMS (Mn = 750). On ajoute ensuite du toluène sec 50% en masse, ainsi que 3,3 ml du catalyseur $H_2PtCl_6$ ($5.10^{-4}$ mole/fonction SiH). On chauffe le milieu réactionnel à 80°C pendant 8 heures. La solution devient noire et un reflux se produit. A la fin de la réaction, la température du milieu réactionnel revient à l'ambiante. Puis une partie de la solution est précipitée dans un minimum d'éthanol et une partie du brut est caractérisée.

Le dosage des fonctions hydrogénosilanes résiduelles du polymère montre que la fonctionnalisation est quasi-quantitative.

Préparation des polymères hydrophiles selon l'invention.

Les produits polymériques hydrophiles à coeur et transparents, selon l'invention, sont préparés selon un procédé comportant les étapes suivantes :

a) on effectue une diffusion et un gonflement du matériau à base de polymère de silicone réticulé contenant le photoamorceur, par exemple de PDMS, obtenu précédemment dans une solution contenant un monomère hydrophile photopolymérisable, un solvant de gonflement du matériau à base de polymère de silicone réticulé, et de préférence un agent de réticulation; et

b) on effectue une polymérisation du monomère hydrophile par irradiation, et éventuellement de l'agent de réticulation, pour obtenir le produit polymérique hydrophile final.

Il n'existe pas de limitation particulière quant au monomère hydrophile à utiliser, dès lors que celui-ci est soluble dans un solvant de gonflement du matériau à base de polymère de silicone et qu'il est par ailleurs photopolymérisable.
On peut utiliser, en particulier l'acide acrylique ou méthacrylique, les hydroxyalkyl(meth)acrylates tels que le méthacrylate d'hydroxyéthyle (HEMA).
Certains monomères permettant d'éviter des dépôts lipidiques ou protéiques peuvent être incorporés, comme, par exemple, le méthacrylate de glycéryle et des dérivés (meth)acrylates de l'acide glucuronique ou galacturonique.
Dans la solution de gonflement, le rapport $\frac{[\text{monomère hydrophile}]}{[\text{solvant}]}$ est de préférence inférieur à 0,5, et mieux encore, inférieur à 0,2.
Le solvant utilisé doit pouvoir, au moins partiellement, solubiliser le monomère hydrophile et être un agent gonflant du matériau de silicone. Il est de préférence peu volatil. On utilisera, par exemple, le toluène, le cyclohexane, le tétrahydrofuranne,le dodécane ou encore un solvant fluoré.
Le réticulant utilisé doit, comme le monomère hydrophile, être photopolymérisable et au moins partiellement soluble dans le solvant. On utilisera par exemple le diméthacrylate d'éthylèneglycol (EGDMA).
Deux modes de mise en oeuvre préférentiels du procédé de préparation des produits hydrophiles, selon l'invention, vont maintenant être décrits :

- Le premier procédé consiste à réaliser l'étape b) de photopolymérisation en comprimant le disque de matériau à base de polymère réticulé de silicone dans un moule transparent aux UV et correspondant à la zone d'irradiation du photoamorceur.

Les réactions de rétrodiffusion de la solution de gonflement sont alors limitées.

- Le second procédé, préférentiellement utilisé, notamment pour une exploitation industrielle, consiste à irradier le matériau gonflé alors que celui-ci est immergé dans la solution de gonflement.

On constate dans ce cas que l'on obtient des produits polymériques dont le taux d'hydrophilie est plus élevé que dans le procédé de l'art antérieur où le photoamorceur est présent dans la solution de gonflement.
Les produits polymériques hydrophiles obtenus ont des structures finales différentes qui dépendent en particulier de la nature du groupement photoamorceur utilisé et au fait que les groupements photoamorceurs sont ou non fixés à des atomes de silicium du polymère de silicone réticulé de la matrice par des liaisons covalentes, et que l'entité photoamorçante formée lors de la photopolymérisation reste fixée ou non au polymère de silicone de la matrice.
Lorsque l'entité photoamorçante reste fixée chimiquement au polymère de silicone de la matrice, le polymère résultant de la photopolymérisation du monomère hydrophile se trouve, au moins partiellement, greffé sur ce polymère de silicone.
En général, lorsque les groupements photoamorceurs ne sont pas greffés au polymère de silicone de la matrice ou lorsque les entités photoamorçantes créées lors de la photopolymérisation sont libres, les produits polymériques hydrophiles obtenus présentent une structure principalement et parfois même uniquement de type IPN.
Ainsi, lorsqu'on utilise comme photoamorceur le vinylsilane d'Irgacure® , ce photoamorceur se greffe par l'intermédiaire des fonctions vinyle sur le PDMS réticulé de la matrice lors de la réticulation. Durant l'irradiation, le photoamorceur forme un radical libre $C(Me_2)OH$ cependant que l'entité photoamorçante

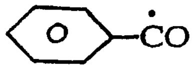

reste fixée au PDMS réticulé de la matrice. De ce fait, le polymère hydrophile résultant de la photopolymérisation du monomère hydrophile, par exemple l'acide acrylique, sera, au moins partiellement, greffé au PDMS réticulé de la matrice par l'intermédiaire des entités photoamorçantes greffées à ce PDMS réticulé.

L'invention concerne donc également un produit polymérique hydrophile obtenu par le procédé décrit ci-dessus, dans lequel des groupements photoamorceurs sont fixés par des liaisons covalentes à des atomes de Si du polymère de silicone réticulé de la matrice et le polymère hydrophile obtenu par photopolymérisation du monomère hydrophile photopolymérisable est, au moins partiellement, greffé sur le polymère de silicone réticulé de la matrice.

Les produits polymériques hydrophiles sont particulièrement adaptés à la fabrication d'articles ophtalmiques tels que des lentilles de contact.

## EXEMPLES

**I** - Préparation de disques ou matériau à base de polymères de silicone réticulés intégrant le photoamorceur.

On prépare des disques silicones (intégrant le photoamorceur) de 14 mm de diamètre et de 0,2 mm d'épaisseur.

Le photoamorceur est ajouté au mélange de prépolymères siloxaniques (10% en poids huile A + 1% en poids huile B) mentionné précédemment.

Le mélange est dégazé et coulé dans

- soit des moules en polyamide (mPA)
- soit des moules en polypropylène (mPP).

Les moules sont ensuite chauffés sous pression pendant 8 heures à 64°C et 15 heures à 83°C.

Les disques obtenus sont transparents, souples et élastiques.

On extrait ensuite les disques obtenus avec du cyclohexane, de façon à éliminer les constituants n'ayant pas réagi.

On procède ensuite à une caractérisation des disques après extraction en vérifiant par un spectre UV de l'échantillon la présence des bandes d'absorption caractéristiques du photoamorceur.

Dans tous les cas, on a constaté que le photoamorceur était bien immobilisé au sein de la matrice en polymère de silicone réticulé des disques.

On a testé les photoamorceurs suivants :

- Méthacrylate de Darocure®
- VSI
- VSI - PDMS - VSI
- ALBP
- ALOBP

**II** - Préparation des produits polymériques hydrophiles.

**II.1 -** Procédé par moulage.

Les disques obtenus précédemment sont gonflés dans une solution contenant :

- le monomère hydrophile
- le solvant mixte du monomère hydrophile et du matériau de silicone
- le réticulant EGDMA.

Quand l'équilibre de diffusion est atteint, l'échantillon est essuyé et placé entre deux parties de moule en quartz (pour éviter la rétrodiffusion de solvant et de monomère) recouvertes d'un film de polyéthylène (afin d'éviter l'adhérence du polymère hydrophile, en particulier le poly(acide acrylique) sur le verre).

La source d'irradiation est une lampe à vapeur de mercure (100 W) maintenue à une distance de 12 à 13 cm.

Une rotation continue de l'échantillon permet d'irradier alternativement chaque face du disque.

L'irradiation est maintenue pendant 15 minutes.
Après irradiation, l'échantillon est séché afin d'éliminer le solvant. Celui-ci est alors extrait à l'eau afin d'éliminer le monomère et les oligomères.
L'échantillon est alors hydraté jusqu'à l'équilibre dans l'eau distillée.

**II.2 -** Procédé en cuve.

Le disque en matière de silicone est introduit dans une cuve en quartz de 0,4 mm d'épaisseur, contenant le monomère hydrophile, le réticulant et le solvant.
L'irradiation est effectuée après gonflement et obtention de l'équilibre de diffusion.
L'irradiation dure 30 minutes. La distance source d'irradiation-échantillon est de 12 à 13 cm.

**EXEMPLES 1 à 16**

Moules de quartz - monomère hydrophile acide acrylique.

Solution de gonflement :

0,294 g d'acide acrylique (AA) ;
0,006 g d'éthylèneglycol diméthacrylate (EGDMA) ;
1,7 g de cyclohexane ;
0,007 g de coamorceur DMABE.

$$\text{rapport} = \frac{[\text{acide acrylique}]}{[\text{cyclohexane}]} = \frac{85}{15}$$

| Ex. n° | Matériau à base de polymère de silicone réticulé | Moule | % PAA | % $H_2O$ |
|---|---|---|---|---|
| 1 | PDMS+1% en poids ALOBP | mPA | 25 | 38 |
| 2 | PDMS+1% en poids ALOBP | mPA | 23 | 32 |
| 3 | PDMS+1% en poids ALOBP | mPA | 26 | 49 |
| 4 | PDMS+1% en poids ALOBP | mPA | 25 | 40 |
| 5 | PDMS+1% en poids ALOBP | mPA | 20 | 43 |
| 6 | PDMS+0,5% en poids ALBP | mPA | 5 | 22 |
| 7 | PDMS+1% en poids ALBP | mPA | 29 | 40 |
| 8 | PDMS+1% en poids ALBP | mPP | 26 | 46 |
| 9 | PDMS+1% en poids ALBP | mPP | 17 | 31 |
| 10 | PDMS+3% en poids ALBP | mPA | 21 | 34 |

Cuve - monomère hydrophile acide acrylique

Solution de gonflement :

0,294 g d'acide acrylique (AA) ;
0,006 g d'EGDMA ;
1,7 g de cyclohexane ;
0,015 g de DMABE.

$$\text{rapport} = \frac{[\text{acide acrylique}]}{[\text{cyclohexane}]} = \frac{85}{15}$$

| Ex. n° | Matériau à base de polymère de silicone réticulé | Moule | % PAA | % $H_2O$ |
|---|---|---|---|---|
| 11 | PDMS+1% en poids ALOBP | mPA | 42 | 58 |
| 12 | PDMS+1% en poids ALOBP | mPA | 22 | 47 |

(suite)

| Ex. n° | Matériau à base de polymère de silicone réticulé | Moule | % PAA | % $H_2O$ |
|---|---|---|---|---|
| 13 | PDMS+1% en poids ALOBP | mPA | 29 | 57 |
| 14 | PDMS+1% en poids ALOBP | mPA | 44 | 47 |
| 15 | PDMS+1% en poids ALOBP | mPA | 25 | 31 |
| 16 | PDMS+1% en poids ALBP | mPP | 33 | 40 |

**EXEMPLES 17 à 26**

**ESSAIS AVEC DIFFERENTS MONOMERES HYDROPHILES**

La solution de gonflement est identique à celle utilisée pour les exemples 1 à 16.

**a)** Photopolymérisation dans un moule en quartz - Matériau à base de polymère de silicone réticulé : PDMS + 1% ALOBP.

| Ex. n° | Monomère hydrophile | Moule | % polymère | % $H_2O$ | % gonflement |
|---|---|---|---|---|---|
| 17 | HEMA | mPP | 5 | 10 | 51 |
| 18 | HEMA | mPA | 9 | 19 | 47 |
| 19 | Acide méthacrylique (AM) | mPP | 31 | 30 | 71 |
| 20 | Acide méthacrylique (AM) | mPA | 24 | 22 | 64 |
| 21 | AA | mPP | 31 | 37 | 74 |
| 22 | AA | mPA | 22 | 29 | 61 |

**b)** Photopolymérisation en cuves - Monomère hydrophile HEMA en solution dans le toluène.

| Ex. n° | Monomère hydrophile | Matériau à base de polymère de silicone réticulé | Moule | % polymère | % $H_2O$ |
|---|---|---|---|---|---|
| 23* | HEMA | PDMS+3% ALBP | mPP | 25 | 12 |
| 24** | HEMA | PDMS+3% ALBP | mPP | 60 | 25 |
| 25* | HEMA | PDMS+1% ALBP | mPP | 12 | 7 |
| 26 | HEMA | PDMS+1 % ALBP | mPP | 57 | 21 |

* Echantillons souples et transparents.

** Transparents et légèrement rigides.

On a mesuré les propriétés mécaniques (contrainte et allongement) de produits polymériques hydrophiles à réseau IPN de l'art antérieur avec des produits polymériques hydrophiles selon l'invention.

Les résultats sont indiqués ci-après.

| | Réseau 1 | Réseau 2 | Moule | Contrainte MPa | Allongement % |
|---|---|---|---|---|---|
| Produit art antérieur | PDMS | PAA | mPP | 0,45 ± 0,26 | 62 ± 39 |
| Produit selon l'invention | PDMS + 1% ALOBP | PAA | mPP | 0,73 ± 0,31 | 95 ± 66 |
| Produit selon l'invention | PDMS + 1% ALOBP | PAA | mPA | 2,12 ± 0,13 | 111 ± 11 |
| Produit selon l'invention | PDMS + 1% ALOBP | PMA | mPP | 2,51 ± 0,33 | 163 ± 29 |

**EXEMPLES 27 à 30**

**Essais avec le coamorceur fixé chimiquement au sein de la matrice de silicone.**

Formule chimique du coamorceur :

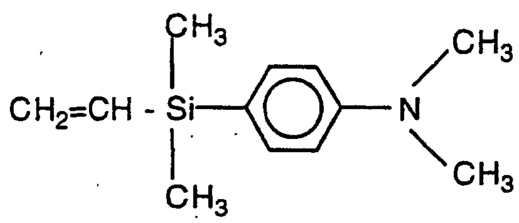

4-diméthylvinylsilane-N,N-diméthylaniline
MM = 205
U.V. 269 nm

Avec le coamorceur fixé chimiquement, le mécanisme d'amorçage sera différent. Dans ce cas, le radical responsable de l'amorçage est le radical issu de l'amine, et donc les chaînes de PAA qui vont se former vont être fixées à la matrice PDMS.

**a) Disques PDMS (moule PP) dans lesquels on a incorporé le coamorceur polymérisable (1%) et ALBP (1%)**.

Solution de gonflement : acide acrylique + réticulant + cyclohexane Polymérisation dans un moule en quartz pendant 15 minutes (sous $N_2$).

| Résultats : | | | |
|---|---|---|---|
| Echantillons PDMS-Coam-ALBP | Gonflement dans la solution % | % PAA | % $H_2O$ |
| Exemple 27 | 71 | 27 | 28 |
| Exemple 28 | 75 | 30 | 30 |

Après photopolymérisation, les échantillons restent transparents et relativement souples.

Le pourcentage de polymère a été obtenu en déterminant initialement la masse m des disques en matériau à base de polymère de silicone réticulé avant l'étape de photopolymérisation.

Après la photopolymérisation, l'échantillon est extrait dans du chloroforme, puis séché et sa masse m' déterminée. D'après les valeurs de m et m', on peut calculer le pourcentage de polymère (par exemple PAA) incorporé :

$$\% \ H_2O = \frac{m_H - m''}{m_H} \times 100.$$

Toutefois, une certaine quantité de polymère peut avoir polymérisé à la surface du disque. Ce polymère peut être éliminé par extraction dans un milieu approprié, par exemple de l'eau distillée dans le cas du PAA. Après déshydratation, la nouvelle masse m" du disque est déterminée, ainsi que la quantité réelle de polymère incorporé et la quantité de polymère polymérisé en surface, extraite.

Les taux d'hydratation sont déterminés après gonflement dans de l'eau distillée ou du sérum physiologique (masse hydratée = $m_H$) d'après la relation :

$$\% \ \text{polymère} = \frac{m' - m}{m'} \times 100$$

## Revendications

1. Procédé de fabrication d'un produit polymérique hydrophile, **caractérisé en ce qu'**il consiste à :

    (a) faire gonfler un matériau comprenant une matrice de polymère de silicone réticulé et des groupements photoamorceurs dispersés et fixés au sein de la matrice dans une solution de gonflement comprenant un solvant de gonflement du polymère de silicone réticulé de la matrice du matériau, un monomère hydrophile photopolymérisable, et éventuellement :

        1) un agent de réticulation ; et
        2) un composé co-amorceur donneur de protons ou d'électrons lorsque le matériau à base de polymère de silicone réticulé comprend des groupements photoamorceurs photoactivables et ne comprend pas de groupements co-amorceurs donneurs de protons ou d'électrons ;

    (b) faire diffuser le monomère hydrophile photopolymérisable dans le matériau gonflé ; et
    (c) polymériser par irradiation. le monomère hydrophile photopolymérisable.

2. Procédé selon la revendication 1, **caractérisée en ce que** le monomère hydrophile photopolymérisable est choisi parmi l'acide acrylique, l'acide méthacrylique et les hydroxyalkyl(méth)acrylates.

3. Procédé selon la revendication 1 ou 2, **caractérisé en ce que** le rapport monomère hydrophile / solvant est inférieur à 0,5.

4. Procédé selon la revendication 1 ou 2, **caractérisé en ce que** le rapport monomère hydrophile / solvant est inférieur à 0,2.

5. Procédé selon l'une quelconque des revendications 1 à 4, **caractérisé en ce que** le solvant est choisi parmi le toluène, le cyclohexane, le dodécane, le tétrahydrofuranne et les solvants fluorés.

6. Procédé selon l'une quelconque des revendications 1 à 5, **caractérisé en ce que** la polymérisation du matériau gonflé s'effectue hors de la solution de gonflement.

7. Procédé selon l'une quelconque des revendications 1 à 5, **caractérisé en ce que** la polymérisation du matériau gonflé s'effectue avec la matériau immergé dans la solution de gonflement.

8. Procédé selon l'une quelconque des revendications 1 à 7 **caractérisé en ce que** les groupements photoamorceurs sont fixés sur le polymère de silicone réticulé constituant la matrice par des liaisons covalentes Si-C.

9. Procédé selon l'une quelconque des revendications 1 à 7 **caractérisé en ce que** les groupements photoamorceurs sont portés par un composé à longue chaîne dispersé et physiquement immobilisé au sein de la matrice.

10. Procédé selon la revendication 9 **caractérisé en ce que** le composé à longue chaîne est un polydiméthylsiloxane (PDMS).

11. Procédé selon l'une quelconque des revendications précédentes, **caractérisé en ce que** les groupements photoamorceurs sont des groupements photoclivables.

12. Procédé selon l'une quelconque des revendications 1 à 10 **caractérisé en ce que** les groupements photoamorceurs sont des groupements photoactivables.

13. Procédé selon la revendication 12 **caractérisé en ce qu'**il comprend en outre des groupements coamorceurs donneurs de protons ou d'électrons.

14. Procédé selon la revendication 13 **caractérisé en ce que** les groupements coamorceurs sont fixés chimiquement sur le polymère de silicone réticulé de la matrice.

15. Procédé selon la revendication 13 **caractérisé en ce que** les groupements coamorceurs sont portés par un composé à longue chaîne dispersé et immobilisé au sein de la matrice.

**16.** Procédé selon l'une quelconque des revendications précédentes, **caractérisé en ce que** le polymère de silicone réticulé de la matrice est un polydiméthylsiloxane.

**17.** Procédé selon la revendication 16 **caractérisé en ce que** le polymère de silicone réticulé de la matrice résulte de la polymérisation thermique d'un mélange d'une huile d'un monomère ou d'un oligomère de polysiloxane porteur de groupements Si-vinyle et d'une huile d'un monomère ou d'un oligomère de polysiloxane porteur de groupements Si-H en présence d'un catalyseur d'hydrosilylation.

**18.** Procédé selon la revendication 12 **caractérisé en ce que** le mélange des huiles de polysiloxane comprend 0,8 à 1,9 liaisons Si-H pour 1 liaison Si-vinyle.

**19.** Produit polymérique hydrophile obtenu par le procédé selon la revendication 1, dans lequel des groupements photoamorceurs sont fixés par des liaisons covalentes à des atomes de silicium du polymère de silicone réticulé de la matrice, et **caractérisé en ce que** le polymère hydrophile obtenu après photopolymérisation du monomère hydrophile polymérisable est, au moins partiellement, greffé sur le polymère de silicone réticulé de la matrice.

**20.** Photoamorceur fonctionnalisé, **caractérisé en ce qu'**il répond à la formule (I):

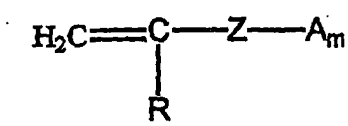

dans laquelle :
R représente un atome d'hydrogène ou un groupe méthyle, Z est une chaîne hydrocarbonée divalente comportant de 1 à 10 atomes de carbone, qui peut être interrompue par 1 à 3 atomes choisis parmi - O - et

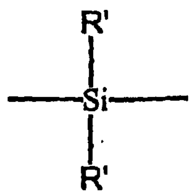

ou R' est indépendamment un atome d'hydrogène ou un groupe alkyle en $C_1$-$C_6$,
et
$A_m$ est un radical choisi parmi les radicaux de formules :

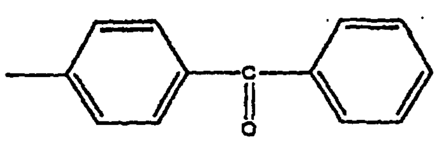

(II)

à condition que Z comporte au moins deux atomes de carbone ;

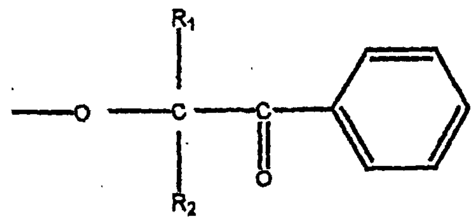

(III) ;

et

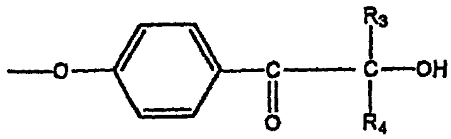

(IV)

dans lesquelles $R_1$, $R_2$, $R_3$ et $R_4$, identiques ou différents, représentent un atome d'hydrogène ou un radical alkyle en $C_1$-$C_6$.

**21.** Photoamorceurs selon la revendication 20 **caractérisés en ce que** Z est une chaîne divalente choisie dans le groupe constitué par :

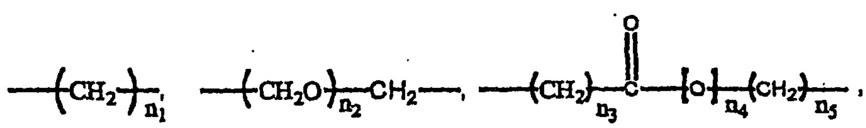

et

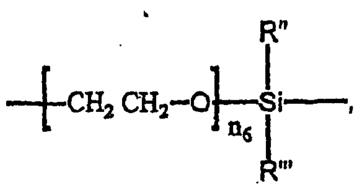

dans lesquelles R" et R"' représentent indépendamment l'un de l'autre un groupe alkyle en $C_1$-$C_6$, $n_1$ et $n_2$ sont des entiers de 1 à 6, $n_3$ et $n_5$ sont des entiers de 0 à 4, $n_4$ est égal à 0 ou 1, et $n_6$ est un entier de 0 à 5.

**22.** Photoamorceur répondant à la formule :

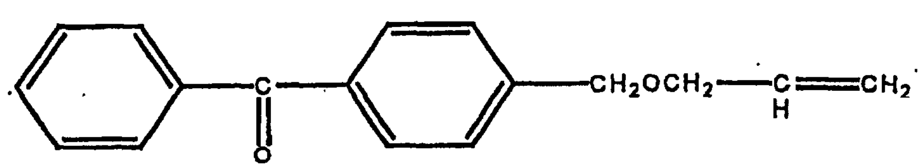

**23.** Photoamorceurs répondant aux formules :

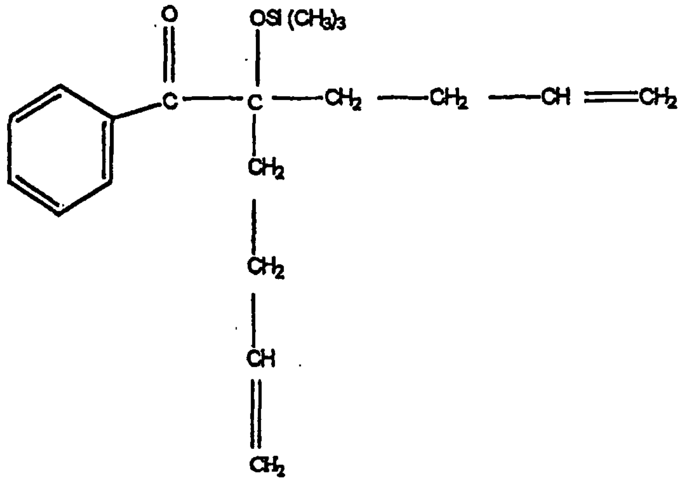

(Vb)

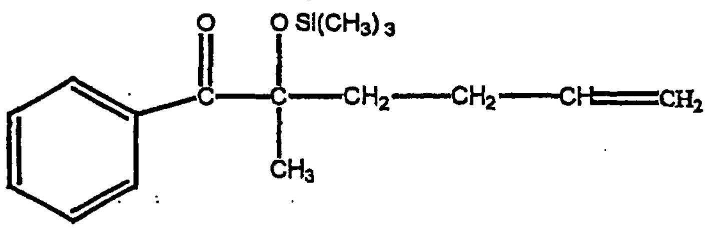

(Vc)

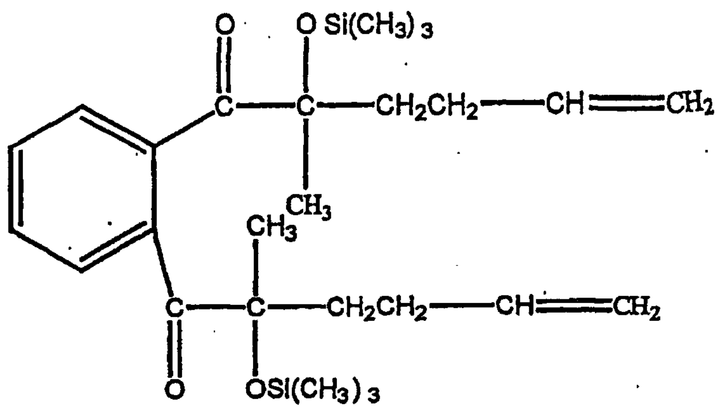

(Vd)

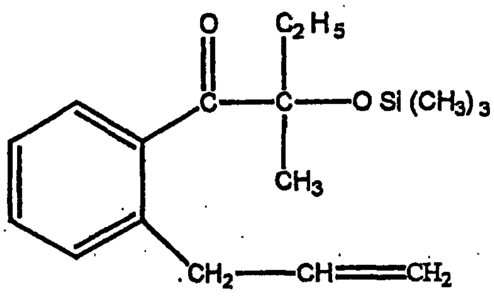

(Ve)

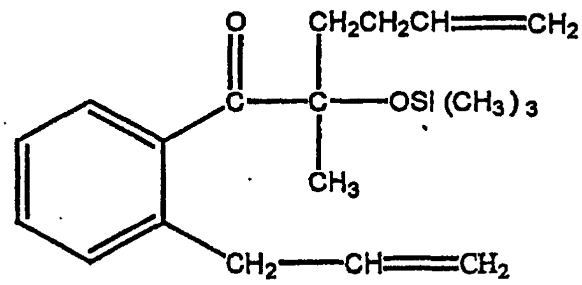

(Vf)

**24.** Macrophotoamorceurs, **caractérisés par le fait qu'**ils résultent de la réaction entre un hydrogénodiméthylpolysiloxane et un composé selon l'une quelconque des revendications 20 à 23.

**Patentansprüche**

**1.** Verfahren zur Herstellung eines hydrophilen Polymerprodukts, **dadurch gekennzeichnet, dass** es aus den folgenden Schritten besteht:

(a) Aufquellen lassen eines Materials, das eine Polymermatrix aus vernetztem Silikon sowie Photoinitiatorgruppen, die in der Matrix dispergiert und fixiert sind, umfasst, in einer Aufquellungslösung, die ein Aufquellungslösungsmittel des vernetzten Silikonpolymers der Matrix des Materials, ein photopolymerisierbares hydrophiles Monomer sowie gegebenenfalls folgendes umfasst:

1) ein Vernetzungsmittel; und

2) eine protonen- oder elektronenabgebende Coinitiatorverbindung, wenn das Material auf der Basis des vernetzten Siliconpolymers photoaktivierbare Photoinitiatorgruppen umfasst und keine protonen- oder elektronenabgebende Coinitiatorgruppen umfasst;

(b) Diffundieren lassen des photopolymerisierbaren hydrophilen Monomers im aufgequollenen Material; und

(c) Polymerisieren des photopolymerisierbaren hydrophilen Monomers durch Bestrahlung.

**2.** Verfahren gemäß Anspruch 1, **dadurch gekennzeichnet, dass** das photopolymerisierbare hydrophile Monomer aus Acrylsäure, Methacrylsäure und Hydroxyalkyl(meth)acrylaten ausgewählt ist.

**3.** Verfahren gemäß Anspruch 1 oder 2, **dadurch gekennzeichnet, dass** das Verhältnis von hydrophilem Monomer/Lösungsmittel unter 0,5 liegt.

**4.** Verfahren gemäß Anspruch 1 oder 2, **dadurch gekennzeichnet, dass** das Verhältnis von hydrophilem Monomer/Lösungsmlttel unter 0,2 liegt.

**5.** Verfahren gemäß einem der Ansprüche 1 bis 4, **dadurch gekennzeichnet, dass** das Lösungsmittel aus Toluol, Cyclohexan, Dodecan, Tetrahydrofuran und fluorierten Lösungsmitteln ausgewählt ist.

**6.** Verfahren gemäß einem der Ansprüche 1 bis 5, **dadurch gekennzeichnet, dass** die Polymerisation des aufgequollenen Materials aus der Aufquellungslösung heraus erfolgt.

**7.** Verfahren gemäß einem der Ansprüche 1 bis 5, **dadurch gekennzeichnet, dass** die Polymerisation des aufgequollenen Materials erfolgt, während das Material in das Aufquellungslösungsmittel eingetaucht ist.

**8.** Verfahren gemäß einem der Ansprüche 1 bis 7, **dadurch gekennzeichnet, dass** die Photoinitiatorgruppen durch kovalente Si-C-Bindungen auf dem vernetzten Silikonpolymer fixiert sind, das die Matrix bildet.

**9.** Verfahren gemäß einem der Ansprüche 1 bis 7, **dadurch gekennzeichnet, dass** sich die Photoinitiatorgruppen an einer langkettigen Verbindung befinden, die in der Matrix dispergiert und physikalisch immobilisiert ist.

**10.** Verfahren gemäß Anspruch 9, **dadurch gekennzeichnet, dass** die langkettige Verbindung ein Polydimethylsiloxan (PDMS) ist.

**11.** Verfahren gemäß einem der vorstehenden Ansprüche, **dadurch gekennzeichnet, dass** die Photoinitiatorgruppen photochemisch abspaltbare Gruppen sind.

**12.** Verfahren gemäß einem der Ansprüche 1 bis 10, **dadurch gekennzeichnet, dass** die Photoinitiatorgruppen photoaktivierbare Gruppen sind.

**13.** Verfahren gemäß Anspruch 12, **dadurch gekennzeichnet, dass** es außerdem protonen- oder elektronenabgebende Coinitiatorgruppen umfasst.

**14.** Verfahren gemäß Anspruch 13, **dadurch gekennzeichnet, dass** die Coinitiatorgruppen chemisch auf dem vernetzten Silikonpolymer der Matrix fixiert sind.

**15.** Verfahren gemäß Anspruch 13, **dadurch gekennzeichnet, dass** sich die Coinitiatorgruppen an einer langkettigen Verbindung befinden, die in der Matrix dispergiert und immobilisiert ist.

**16.** Verfahren gemäß einem der vorstehenden Ansprüche, **dadurch gekennzeichnet, dass** das vernetzte Silikonpolymer der Matrix ein Polydimethylsiloxan ist.

**17.** Verfahren gemäß Anspruch 16, **dadurch gekennzeichnet, dass** das vernetzte Silikonpolymer der Matrix aus der thermischen Polymerisation eines Gemischs aus einem Öl eines Polysiloxanmonomers oder -oligomers, das Si-Vinyl-Gruppen trägt, und einem Öl eines Polysiloxanmonomers oder -oligomers, das Si-H-Gruppen trägt, in Gegenwart eines Hydrosilylierungskatalysators stammt.

**18.** Verfahren gemäß Anspruch 12, **dadurch gekennzeichnet, dass** das Gemisch der Polysiloxanöle 0,8 bis 1,9 Si-H-Bindungen pro Si-Vinyl-Bindung umfasst.

**19.** Hydrophiles Polymerprodukt, das nach dem Verfahren gemäß Anspruch 1 erhalten wurde, wobei die Photoinitiatorgruppen durch Bindungen an Siliciumatomen des vernetzten Silikonpolymers der Matrix fixiert sind, **dadurch gekennzeichnet, dass** das erhaltene hydrophile Polymer nach der Photopolymerisation des polymerisierbaren hydrophilen Monomers wenigstens partiell auf das vernetzte Silikonpolymer der Matrix aufgepfropft wird.

**20.** Funktionalisierter Photoinitiator, **dadurch gekennzeichnet, dass** er der Formel (I)

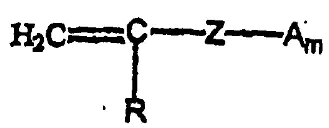

entspricht, wobei:
R ein Wasserstoffatom oder eine Methylgruppe darstellt, Z eine zweiwertige Kohlenwasserstoffkette mit 1 bis 10 Kohlenstoffatomen ist, welche durch 1 bis 3 Atome unterbrochen sein kann, die ausgewählt sind aus -O- und

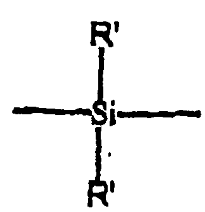

,

wobei R' unabhängig ein Wasserstoffatom oder eine $C_1$-$C_6$-Alkylgruppe ist; und

$A_m$ ein Rest ist, der aus Resten der folgenden Formeln ausgewählt ist:

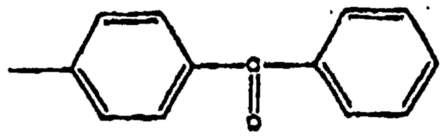

(II)

mit der Maßgabe, dass Z wenigstens zwei Kohlenstoffatome umfasst;

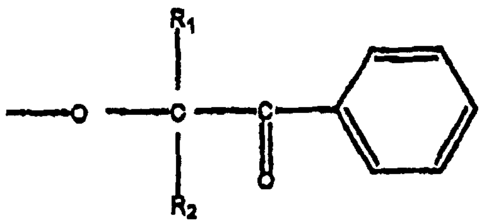

(III);

und

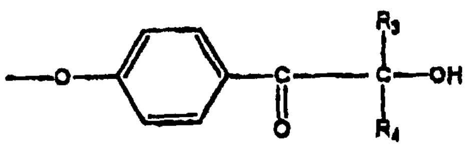

(IV)

wobei $R_1$, $R_2$, $R_3$ und $R_4$ gleich oder verschieden sind und ein Wasserstoffatom oder einen $C_1$-$C_6$-Alkylrest darstellen.

**21.** Photoinitiatoren gemäß Anspruch 20, **dadurch gekennzeichnet, dass** Z eine zweiwertige Kette ist, die aus der folgenden Gruppe ausgewählt ist:

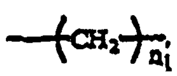 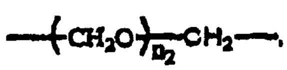 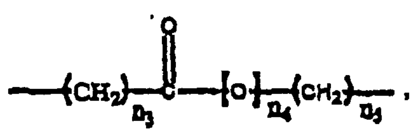

und

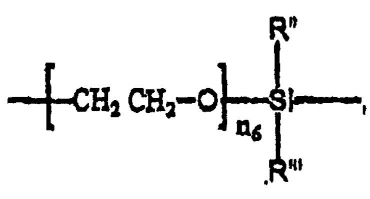

wobei R" und R"' unabhängig voneinander eine $C_1$-$C_6$-Alkylgruppe darstellen, $n_1$ und $n_2$ ganze Zahlen von 1 bis 6 sind, $n_3$ und $n_5$ ganze Zahlen von 0 bis 4 sind, $n_4$ gleich 0 oder 1 ist und $n_6$ eine ganze Zahl von 0 bis 5 ist.

**22.** Photoinitiator, der der folgenden Formel entspricht:

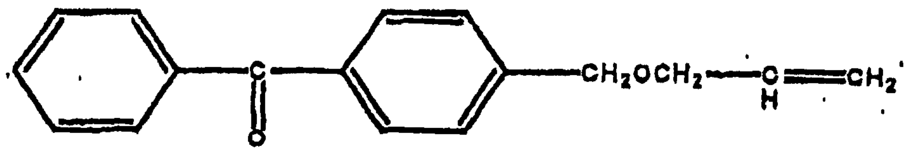

**23.** Photoinitiatoren, die den folgenden Formeln entsprechen:

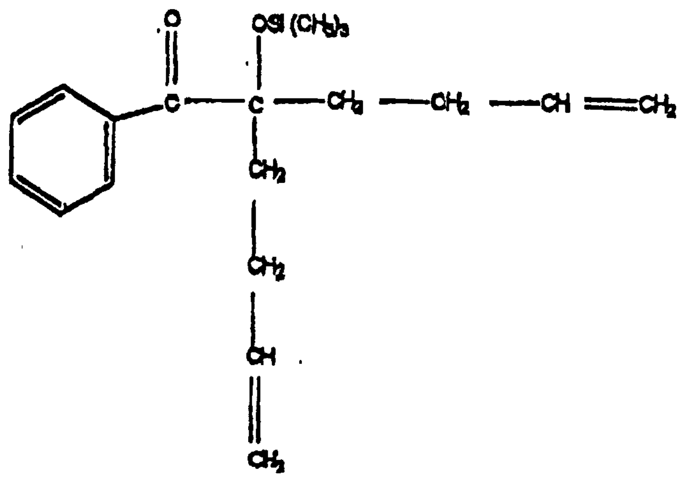

(Vb)

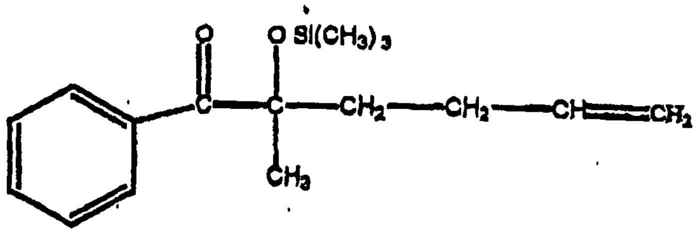

(Vc)

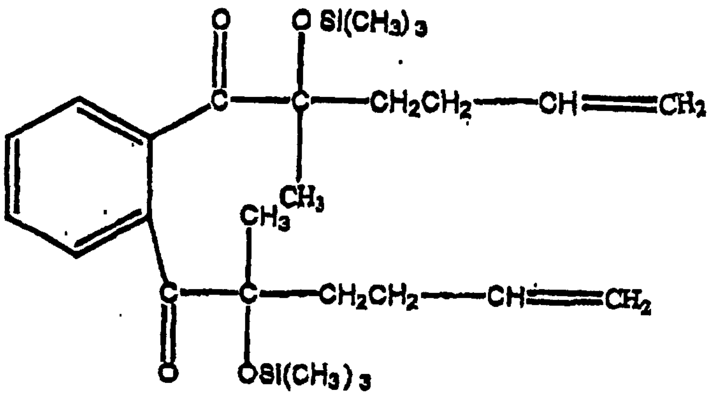

(Vd)

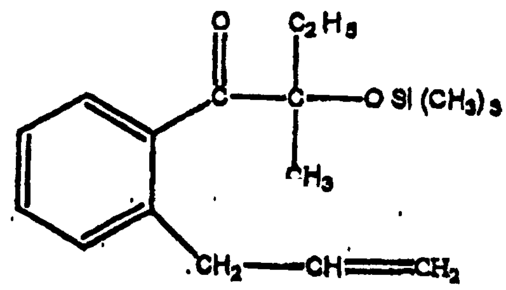

(Ve)

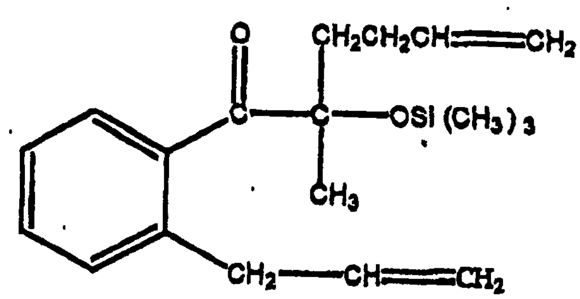

(Vf)

**24.** Makrophotoinitiatoren, **dadurch gekennzeichnet, dass** sie aus der Reaktion zwischen einem Hydrogendimethylpolysiloxan und einer Verbindung gemäß einem der Ansprüche 20 bis 23 stammen.

## Claims

**1.** Method of producing a hydrophilic polymeric product, **characterized in that** it comprises :

(a) causing a material comprising a crosslinked silicone polymer matrix and photoinitiator groups dispersed and fixed within the matrix to swell in a swelling solution comprising a solvent for swelling the crosslinked silicone polymer of the material of the matrix, a photopolymerizable hydrophilic monomer, and optionally :

1) a crosslinking agent; and
2) a proton or electron donor co-initiator compound when the crosslinked silicone polymer-based material contains photoactivable photoinitiator groups and does not contain proton or electron donor co-initiator groups ;

(b) causing the photopolymerizable hydrophilic monomer to diffuse into the swelled material ; and
(c) polymerizing the photopolymerizable hydrophilic monomer by irradiation.

**2.** Method according to claim 1, **characterized in that** the photopolymerizable hydrophilic monomer is selected from acrylic acid, methacrylic acid and the hydroxyalkyl(meth)acrylates.

**3.** Method according to claims 1 or 2, **characterized in that** the hydrophilic monomer/solvent ratio is less than 0.5.

**4.** Method according to claims 1 or 2, **characterized in that** the hydrophilic monomer/solvent ratio is less than 0.2.

**5.** Method according to any one of claims 1 to 4, **characterized in that** the solvent is selected from toluene, cyclohexane, dodecane, tetrahydrofuran and the fluorinated solvents.

**6.** Method according to any one of claims 1 to 5, **characterized in that** the polymerization of the swelled material is performed outside the swelling solution.

**7.** Method according to any one of claims 1 to 5, **characterized in that** the polymerization of the swelled material is

performed with the material immersed in the swelling solution.

**8.** Method according to any one of claims 1 to 7, **characterized in that** the photoinitiator groups are fixed onto the crosslinked silicone polymer comprising the matrix by covalent Si-C bonds.

**9.** Method according to any one of claims 1 to 7, **characterized in that** the photoinitiator groups are carried by a long-chain compound dispersed and physically immobilized within the matrix.

**10.** Method according to claim 9, **characterized in that** the long-chain compound is a polydimethylsiloxane (PDMS).

**11.** Method according to any one of the preceding claims, **characterized in that** the photoinitiator groups are photocleavable groups.

**12.** Method according to any one of claims 1 to 10, **characterized in that** the photoinitiator groups are photoactivable groups.

**13.** Method according to claim 12, **characterized in that** it further comprises proton or electron donor co-initiator groups.

**14.** Method according to claim 13, **characterized in that** the co-initiator groups are chemically fixed onto the crosslinked silicone polymer of the matrix.

**15.** Method according to claim 13, **characterized in that** the co-initiator groups are supported by a long-chain compound dispersed and immobilized within the matrix.

**16.** Method according to any one of the preceding claims, **characterized in that** the crosslinked silicone polymer of the matrix is a polydimethylsiloxane.

**17.** Method according to claim 16, **characterized in that** the crosslinked silicone polymer of the matrix results from the thermal polymerization of a mixture of an oil of a monomer or an oligomer of polysiloxane having Si-vinyl groups and an oil of a monomer or an oligomer of polysiloxane having Si-H groups in the presence of a hydrosilylation catalyst.

**18.** Method according to claim 12, **characterized in that** the mixture of polysiloxane oils contains 0.8 to 1.9 Si-H bonds for 1 Si-vinyl bond.

**19.** Hydrophilic polymeric product obtained by the method according to claim 1, wherein photoinitiator groups are fixed by covalent bonds to the silicon atoms of the crosslinked silicon polymer of the matrix, and **characterized in that** the hydrophilic polymer obtained after photopolymerization of the polymerizable hydrophilic monomer is at least partially grafted onto the crosslinked silicon polymer of the matrix.

**20.** Functionalized photoinitiator, **characterized in that** it has the formula (I) :

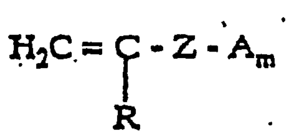

wherein :
R represents a hydrogen atom or a methyl group, Z is a divalent hydrocarbon chain containing from 1 to 10 carbon atoms, into which may be inserted 1 to 3 atoms selected from -O- and

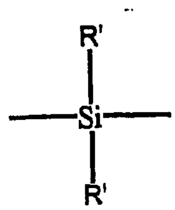

where R' is independently a hydrogen atom or a $C_1$-$C_6$ alkyl group, and

$A_m$ is a group selected from groups of formula :

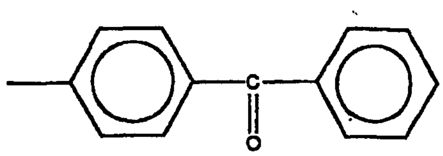 (II)

with the proviso that Z contains at least two carbon atoms ;

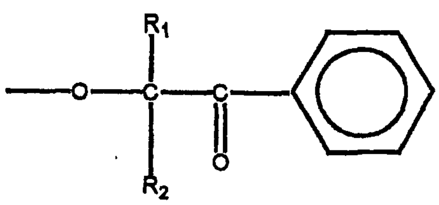 (III) ;

and

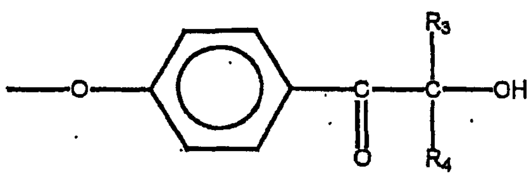 (IV) ;

wherein $R_1$, $R_2$, $R_3$ and $R_4$, identical or different, represent a hydrogen atom or a $C_1$-$C_6$ alkyl group.

**21.** Photoinitiators according to claim 20, **characterized in that** Z is a divalent chain selected from the group comprising :

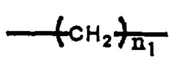 , 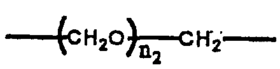 , 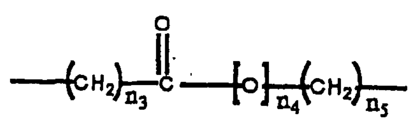

and

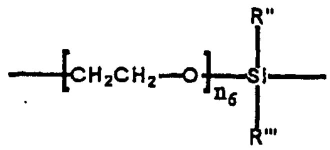

,

wherein R" and R"' independently represent a $C_1$-$C_6$ alkyl group, $n_1$ and $n_2$ are integers from 1 to 6, $n_3$ and $n_5$ are integers from 0 to 4, $n_4$ is equal to 0 or 1, and $n_6$ is an integer from 0 to 5.

**22.** Photoinitiator having the formula :

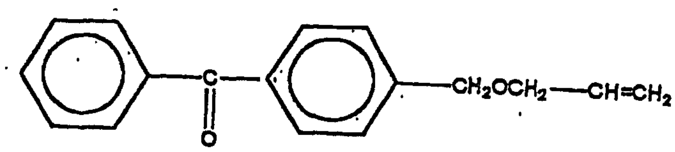

**23.** Photoinitiators having the formulas :

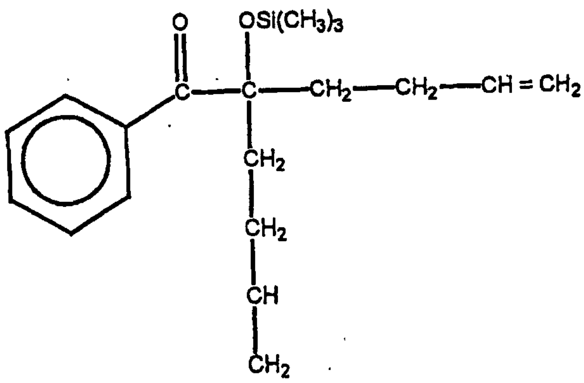

(Vb)

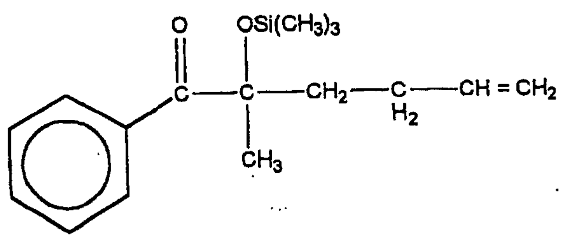

(Vc)

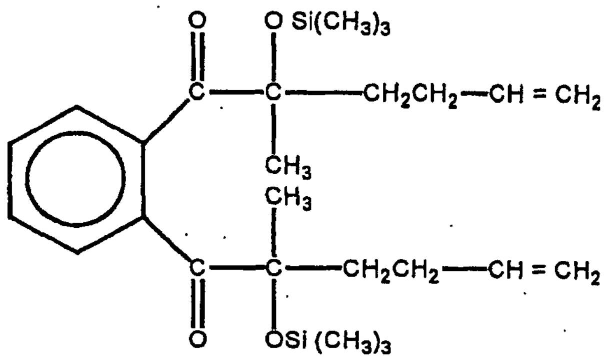

(Vd)

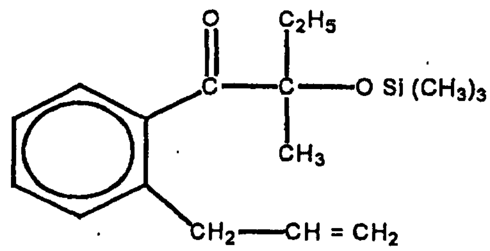

(Ve)

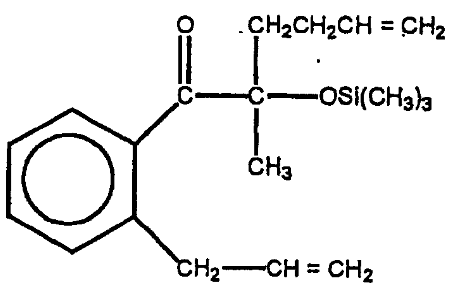

(Vf)

**24.** Macrophotoinitiators, **characterized by** the fact that they result from the reaction between a hydrogenodimethylpolysiloxane and a compound according to any one of claims 20 to 23.